# EUROPEAN PATENT SPECIFICATION

(11) **EP 0 359 480 B1**
(45) Date of publication and mention of the grant of the patent: **30.03.1994**
(21) Application number: 89309115.7
(22) Date of filing: 08.09.1989
(51) Int. Cl.: C07D 487/14, A61K 31/40

(54) **Mitomycin derivatives**
Mitomycinderivate
Dérivés de mitomycine

(30) Priority: 09.09.1988 JP 224484/88
(43) Date of publication of application: 21.03.1990
(73) Proprietor: KYOWA HAKKO KOGYO CO., LTD., Chiyoda-ku, Tokyo (JP)
(72) Inventor: Kanda, Yutaka c/o Patent Department, Chiyoda-ku Tokyo (JP); Kasai, Masaji c/o Patent Department, Chiyoda-ku Tokyo (JP); Arai, Hitoshi c/o Patent Department, Chiyoda-ku Tokyo (JP); Morimoto, Makoto c/o Patent Department, Chiyoda-ku Tokyo (JP); Ashizawa, Tadashi c/o Patent Department, Chiyoda-ku Tokyo (JP)
(74) Representative: Lambert, Hugh Richmond

(56) References cited:
- EP-A- 197 799
- EP-A- 0 284 380
- EP-A- 0 307 179
- PATENT ABSTRACTS OF JAPAN vol. 3, no. 142 (C-65)(161), 24 Nov. 1979; & JP-A- 54 122797
- PATENT ABSTRACTS OF JAPAN, vol. 4, no. 178 (C-34)(660), 10 Dec. 1980; & JP-A-55 118396
- PATENT ABSTRACTS OF JAPAN vol. 4, no. 81 (C-14)(26), 11 June 1980; & JP-A-55 45322 /KYOWA HAKKO KOGYO K.K.) 31.03.1980 (Cat.D)

## Description

The present invention relates to novel mitomycin derivatives having anti-tumour and antibacterial activity.

Several derivatives of mitomycin are known and are known to have anti-tumour and antibacterial activity and are thus of potential utility as anti-tumour agents and as antibiotics. Generally such mitomycins are available from natural sources, although sometimes only in trace amounts. Amongst the known mitomycins from natural sources are:

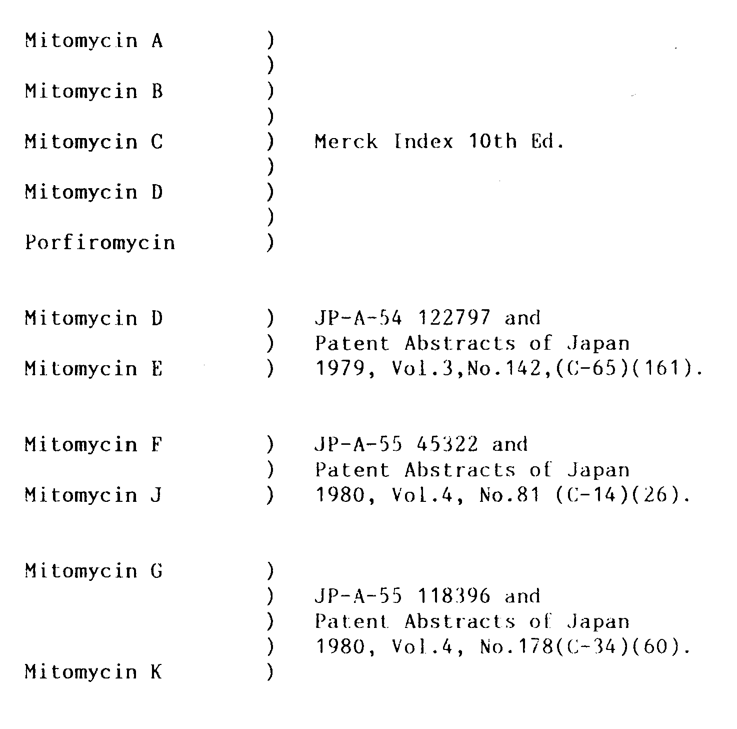

The structures of these known mitomycins obtained from natural sources are shown in Table 1.

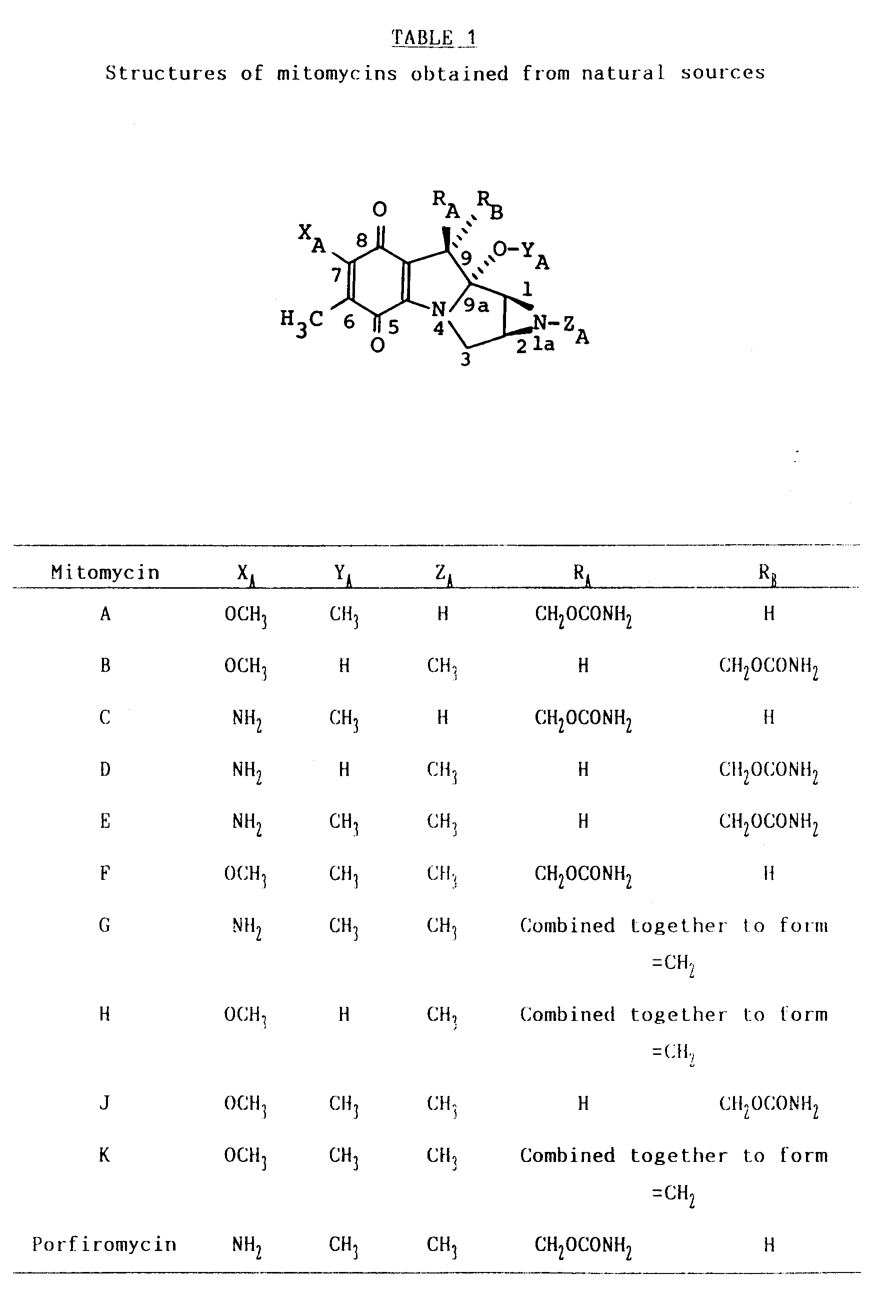

Some of the above mitomycins show an excellent anti-tumour activity, but they have also serious side effects such as decreasing the number of leucocytes. In view of this, other mitomycins have been synthesized in attempts to enhance the activity and/or to alleviate toxicity. Amongst those semi-synthetic mitomycins are those compounds wherein the C-methoxy or amino group has been modified, see inter-alia EP-A-0 197 799 and EP-A-0 284 380, and those where the C₆ methyl substituent has been deuterated or tritiated, see JP-A-10 70490 and the corresponding EP-A-0 307 179.

In accordance with the present invention further semi-synthetic derivatives having anti-tumour and antibacterial activity are provided, closely related to mitomycins F and J, supra, but having a modified methyl substituent in the 6-position.

These are compounds of the formula (I): 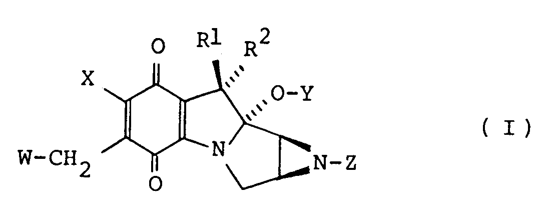
wherein:
W is an RO or RS group, where R is H; straight or branched chain C₁-C₁₂ alkyl, optionally containing one or two substituents selected from: OH, C₁-C₄ alkoxy, C₂-C₅ alkoxycarbonyl, C₁-C₄ alkanoylamino, C₁-C₄ alkanoyloxy, aroylamino or aroyloxy, wherein the aroyl group is benzoyl, toluoyl, p-nitrobenzoyl or naphthoyl, halogen and C₃-C₆ cycloalkyl; C₂-C₁₂ alkenyl, optionally containing one or two substituents selected from: OH, C₁-C₄ alkoxy, C₂-C₅ alkoxycarbonyl, and halogen; phenyl, naphthyl, benzyl, penethyl or benzhydryl, optionally containing from 1-3 substituents in the aryl moiety and selected from: OH, C₁-C₄ alkoxy, C₂-C₅ alkoxycarbonyl, C₁-C₄, alkanoylamino, C₁-C₄, alkanoyloxy, aroylamino or aroyloxy, wherein the aroyl group is benzoyl, toluoyl, p-nitrobenzoyl or naphthoyl, halogen, nitro and C₁-C₄ alkyl;
X is methoxy or amino;
Y is H or methyl;
Z is H, methyl, C₁-C₄ alkanoyl, benzoyl, toluyl, p-nitrobenzoyl, or naphthoyl; and
nitrobenzoyl, or naphthoyl; and
one of R¹ and R² represents carbamoyloxymethyl and the other represents hydrogen, or together they jointly represent a methylene (=CH₂) group;
or wherein:
W and X together form either an -S(CH₂)₂NH- group or a group of the formula: 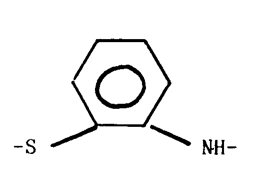
In the definition of W in formula (I), is preferably an RO or RS group wherein the alkyl group (R) is methyl, ethyl, n-propyl, isopropyl, n-butyl, isobutyl, tert-butyl, pentyl, hexyl, heptyl, octyl, decyl or dodecyl. When R is substituted, examples of suitable substituents are hydroxy, methoxy, ethoxy, propoxy or butoxy; methoxycarbonyl, ethoxycarbonyl, propoxycarbonyl or tertbutoxycarbonyl; fluorine, chlorine, bromine or iodine; cyclopropyl, cyclopentyl and cyclohexyl.

When R is alkenyl, suitable examples are vinyl, alkyl, homoallyl, crotyl and cis-7-dodecenyl. When the alkenyl group is substituted suitable substituent(s) are the same as exemplified above for the alkyl substituents.

When R is substituted aryl, the substituents in the aromatic ring may be the same or different and may be, for example, hydroxy, nitro, methyl, ethyl, propyl, isopropyl, butyl, isobutyl or tert-butyl, or as otherwise exemplified above for the alkyl substituents.

Compounds of Formula (I) are obtained by the reaction schemes described below: 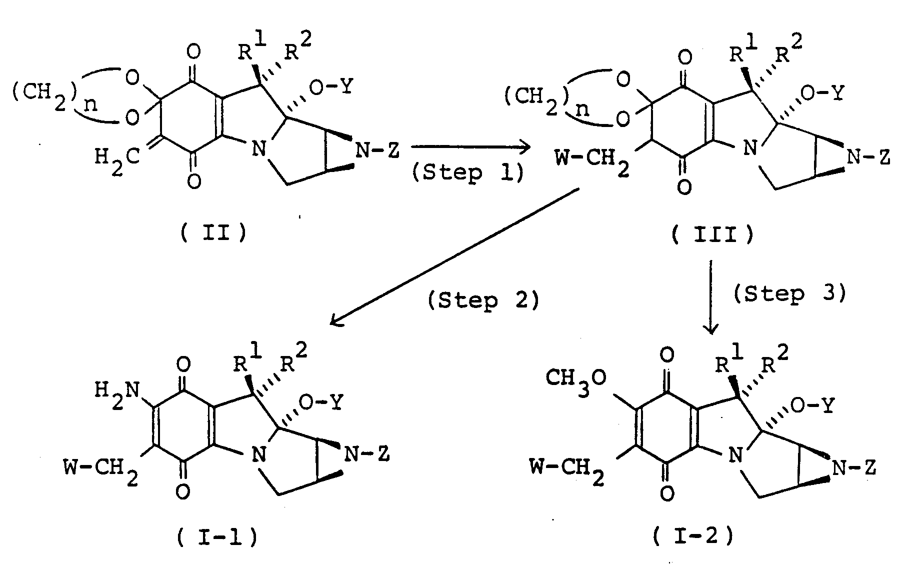
In the formulae, n represents an integer of 2 or 3; and W, Y, Z, R¹ and R² have the same significances as defined above.

### (Step 1)

Compounds (III) can be prepared by subjecting Compounds (II) to reaction with an alcohol (ROH) or a thiol (RSH) (wherein R has the same significance as defined above) in a solvent inert to the reaction, if necessary, in the presence of a base.

As the solvent, ether, tetrahydrofuran, methylene chloride, chloroform, etc. may be used singly or in combination. In the reaction with the alcohol, the alcohol as a reactant may also be used as a solvent. Further, in the reaction with the alcohol, a base may be added to the reaction system. As the base, tetrabutyl ammonium hydroxide, benzyl trimethyl ammonium hydroxide, etc. are preferably used in a catalytic amount.

The reaction is usually completed in a temperature range of 0 to 30°C in 10 minutes to 2 days.

Compounds (II) which are starting materials can be prepared by the following reaction steps. 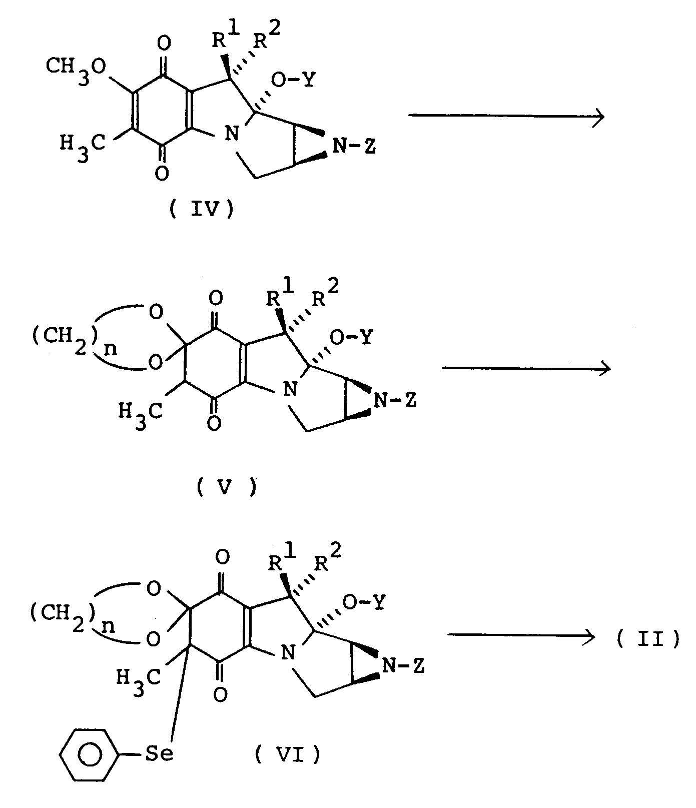
In the formulae, Y, Z, R¹, R² and n have the significances as defined above.

First, mitomycins (IV) having methoxy group at the 7-position (for example, those shown in Table 1) are allowed to react with ehtylene glycol or 1,3-propanediol in the presence of a base to give Compounds (V) disclosed in JP-A-106275 and the corresponding EP-A-0284380.

Next, Compounds (V) are allowed to react with a selenenylating agent such as phenylselenenyl halide to give Compounds (VI). Compounds (VI) are oxidised with an oxidising agent such as metachloroperbenzoic acid to give Compounds (II) disclosed in JP-A-1070490 and the corresponding EP-A-0 307 179.

Compounds (III) can also be prepared directly from Compounds (IV) and the alcohol or thiol under the conditions similar to those described above.

### (Step 2)

Compounds (I-1) [Compounds (I) wherein X is amino] can be prepared by subjecting Compounds (III) obtained in Step 1 to reaction with from an equivalent amount up to a large excess of ammonia or ammonium acetate in an inert solvent. Any solvent is usable so long as it can dissolve Compounds (III). For example, alcohols such as methanol and ethanol; ethers such as diethyl ether and tetrahydrofuran; halogenated hydrocarbons such as methylene chloride and chloroform; acetonitrile, dimethylformamide, dimethylsulfoxide, etc. may be used single or in combination. The reaction is usually completed in a temperature range of 0 to 30°C in one hour to 7 days.

### (Step 3)

Compounds (I-2) [Compounds (I) wherein X is methoxyl] can be prepared by subjecting Compounds (III) to reaction with methanol in the presence of a base. As the base used for the reaction, there may be mentioned R³OM (wherein R³ represents a lower alkyl group having 1 to 4 carbon atoms such as methyl, ethyl, butyl or tert-butyl; and M represents an alkali metal such as lithium, sodium or potassium or an alkaline earth metal such as calcium or magnesium); hydroxides, carbonates or bicarbonates of the same alkali metals or alkaline earth metals as described above; tertiary organic amines such as triethylamine, tributylamine and N-methylmorpholine; quaternary ammonium hydroxides such as tetrabutyl ammonium hydroxide, etc.
An amount of the base is in the range of 0.001 to 10 equivalents, preferably 0.01 to 3 equivalents, based on Compound (III). The reaction is usually completed in a temperature range of 20 to 30°C in 2 to 24 hours.

When Compounds (III) wherein Z is acyl are used in Steps 2 and 3, deacylation sometimes occurs simultaneously under the reaction conditions described above to give the corresponding Compounds (I-1) and (I-2) wherein Z is hydrogen, respectively.

Compounds (I) wherein W and X are combined together to form -S(CH₂)₂NH- or 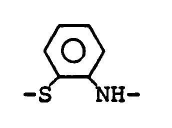
can be prepared by subjecting Compounds (II) to reaction with the corresponding 2-aminoethanethiol or 2-aminothiophenol or hydrochlorides thereof in a similar manner as in Step 1. When the thiol hydrochlorides are used, it is preferred to add organic bases such as pyridine and triethylamine in an equivalent amount or more.

Compounds (I) wherein W is hydroxy can also be prepared by treating Compounds (III) wherein W is 3,4-dimethoxybenzyloxy group with a suitable oxidizing agent, for example, 2,3-dichloro-5,6-dicyano-1,4-benzoquinone (DDQ), and subjecting the resulting Compounds (III) wherein W is hydroxy to Step 2 or 3. As the solvent used for the oxidation, a solvent mixture of water and a water-immiscible solvent such as methylene chloride or chloroform is preferred. The reaction is usually completed in a temperature range of 0 to 30°C in 2 to 12 hours.

Further, Compounds (I) wherein Z is hydrogen can also be prepared by hydrolysis (deacylation) of the corresponding Compounds (I) wherein Z is protected with an acyl such as acetyl under basic conditions. As the base, amines such as ammonia, inorganic bases such as sodium bicarbonate, sodium hydroxide and potassium hydroxide, etc. can be used. It is preferred to use methanol, water, etc. as the solvent. The reaction is usually completed in 1 to 20 hours.

Post-treatments in the respective steps for producing the intermediates and the desired compounds described above are carried out in the following manner. The reaction solution is concentrated as it is or, if necessary, after neutralization, and then purified. Alternatively, after being neutralized if necessary, the reaction solution is extracted with a water-immiscible solvent such as chloroform, methylene chloride or ethyl acetate, and the extract is washed with water, aqueous sodium chloride solution, etc., and then concentrated, followed by purification. Purification is effected by column chromatography using silica gel, etc. or thin layer chromatography (TLC) or by recrystallization, etc. In the case of the intermediates, they may be directly subjected to the subsequent reaction without any particular purification.

Examples of Compounds (I) obtained by the process described above are shown in Table 2.

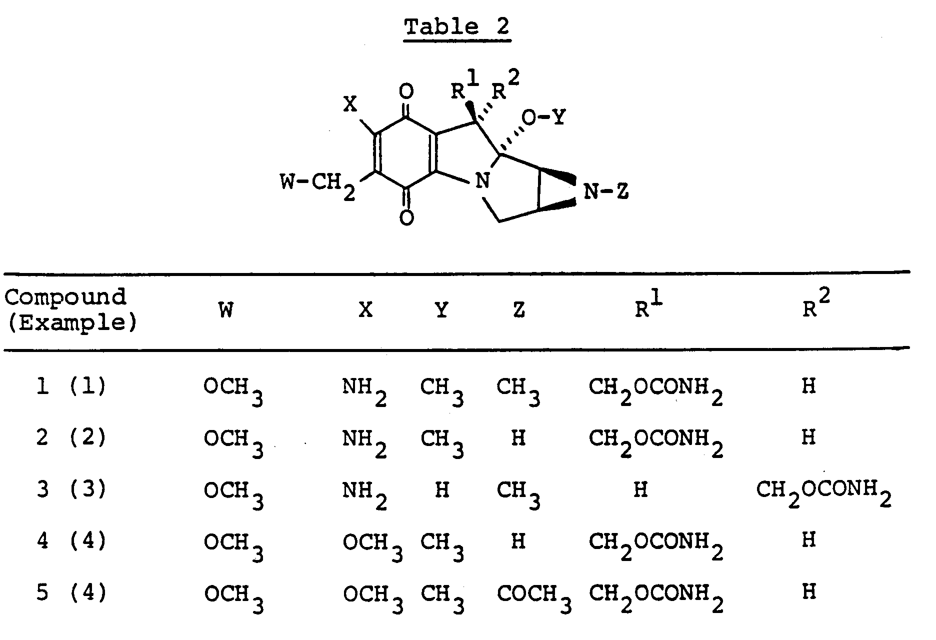

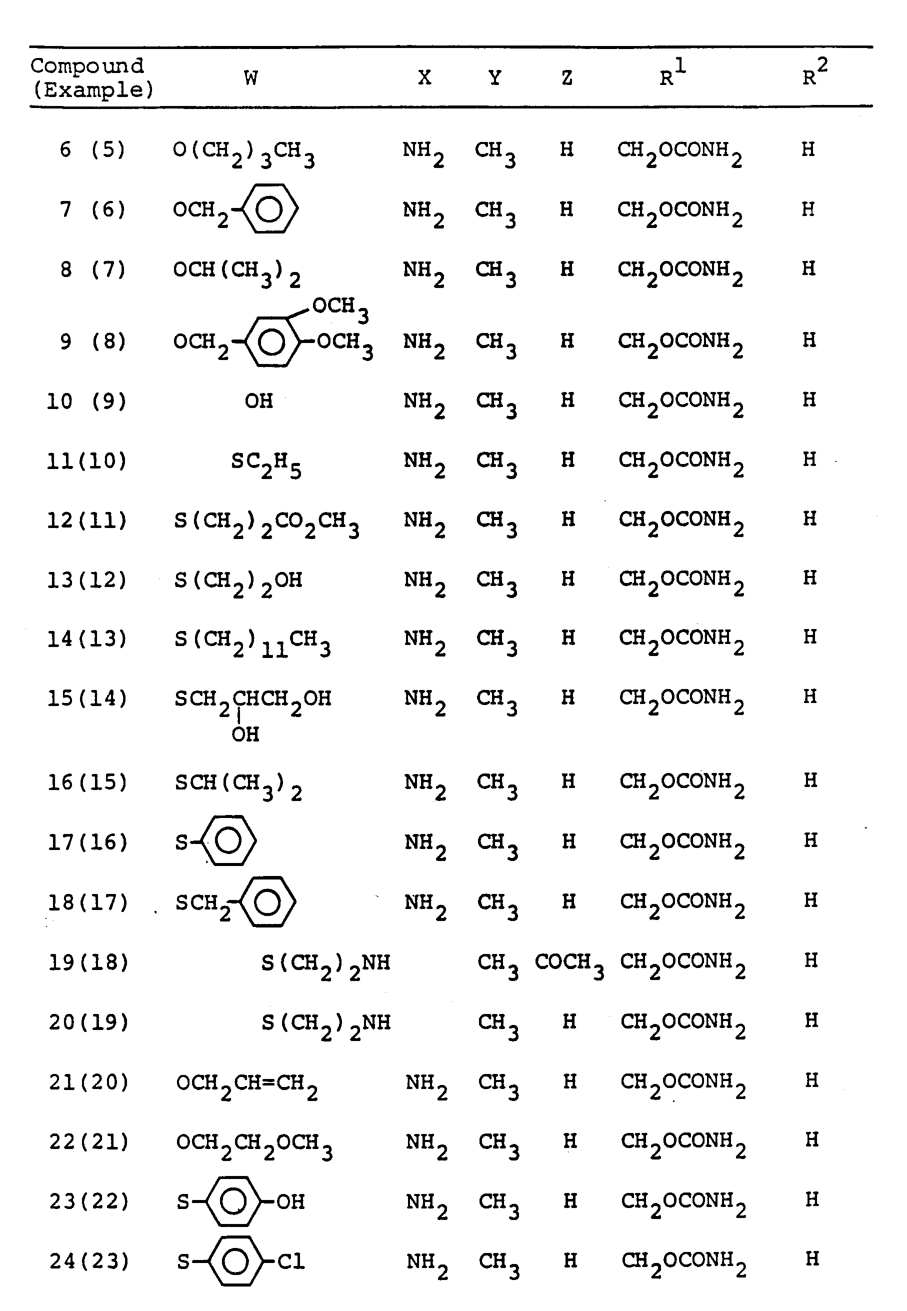

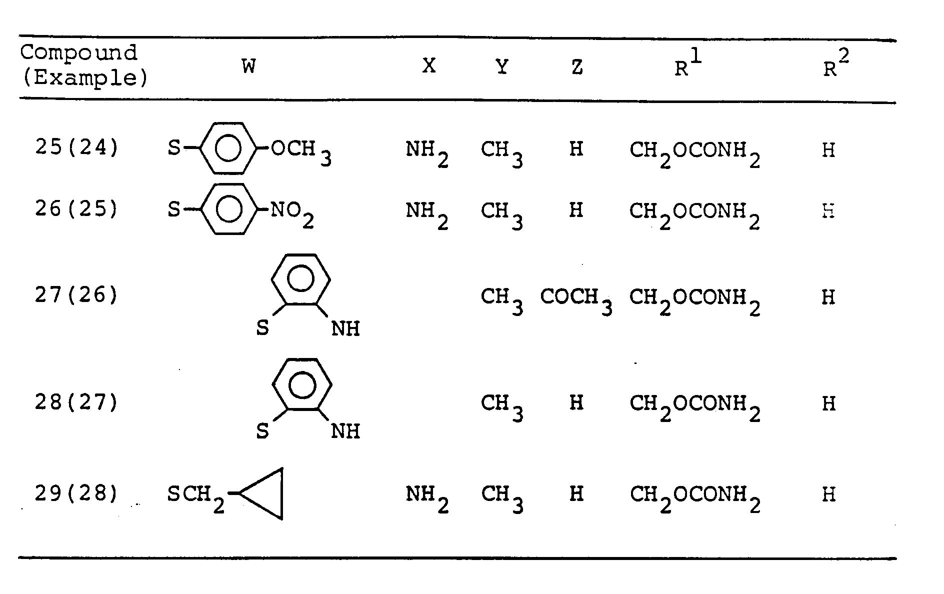

The antibacterial activity and anti-tumor activity of representative Compounds (I) are specifically shown below by referring to experimental examples.

### Experimental Example 1

The antibacterial activity of Compounds (I) against various bacteria [minimum growth inhibitory concentration (µg/ml)] is shown in Table 3.

The minimum growth inhibitory concentration was determined by the agar dilution method at pH 7.0. In the table, names of bacteria are shown by the following symbols.
- SF :: Streptococcus faecium ATCC 10541
- SA :: Staphylococcus aureus ATCC 6538P
- PV :: Proteus vulgaris ATCC 6897
- KP :: Klebsiella pneumoniae ATCC 10031

**Table 3**

| Compound | SF | SA | PV | KP |
|---|---|---|---|---|
| 1 | 0.63 | 0.31 | >40 | 0.63 |
| 2 | 1.3 | 1.3 | 1.3 | 0.31 |
| 4 | 10 | 10 | 2.5 | 0.63 |
| 6 | 0.31 | 0.63 | - | 5.0 |
| 7 | 0.16 | 0.63 | - | 5.0 |
| 8 | 0.31 | 0.63 | 40 | 0.63 |
| 10 | 10 | 5.0 | 5.0 | 1.3 |
| 11 | 0.31 | 0.039 | 0.16 | 1.3 |
| 12 | 0.63 | 0.31 | 1.3 | 0.31 |
| 13 | 10 | 10 | 40 | 5.0 |
| 14 | 0.39 | 0.20 | >50 | - |
| 16 | 0.078 | 0.16 | 10 | 1.3 |
| 17 | 0.31 | 20 | 40 | 10 |
| 18 | 0.039 | 0.16 | 40 | 5.0 |
| 20 | 5.0 | 2.5 | 40 | 10 |

### Experimental Example 2

The effect of representative Compounds (I) against Sarcoma 180 solid tumor is shown in Table 4.

LD₅₀ and ED₅₀ were determined by the following methods, respectively.

### (1) Determination of LD₅₀

Five ddY mice were used for each group, and the test compounds were administered once to the mice intraperitoneally or intravenously. After the administration, the mice were observed for 14 days and deaths were noted. LD₅₀ was calculated from the death rate of each group according to the Behrens Kaerber's method.

### (2) Determination of ED₅₀

Sarcoma 180 cells (5 x10⁶ cells) were implanted intraperitoneally into a ddY mouse and the cells were collected from the ascitic fluid of the animal 7 days after the implantation. The cells were washed once with sterilized physiological saline solution and then suspended in sterilized physiological saline solution to prepare a cell suspension containing 5 x10⁷ cells/ml. The suspension (0.1 ml) was subcutaneously implanted into the right axilla of male ddY mice weighing 20 ± 2 g.

A test compound was dissolved in physiological saline solution or Tween 80-containing physiological saline solution, and 0.1 to 0.2 ml of the solution was administered to 5 mice as one group intraperitoneally or intravenously 24 hours after the implantation of the tumor cells.

The anti-tumor activity of the test compound was determined by measuring the major axis (a) and the minor axis (b) of the tumor 7 days after the implantation, calculating a value of (a) x (b)²/2 corresponding to the volume of the tumor, and calculating a ratio T/C of the volume (T) of the group which received the test compound to the volume (C) of the control group which received no test compound. T/C at each dose given was plotted on a graph in which T/C is shown by an ordinary scale on the vertical axis and a dose is shown by a logarithmic scale on the abscissa.
The relationship between the dose and T/C was determined to be a straight line by the least-squares method. According to the regression equation of the straight line obtained, a dose at which T/C showed 0.5 was calculated as ED₅₀.

**Table 4**

| Compound | LD₅₀ (mg/kg) | ED₅₀ (mg/kg) | Route for Administration |
|---|---|---|---|
| 2 | 75 | 34.0 | intraperitoneal |
| 6 | >50 | 38.0 | intraperitoneal |
| 7 | 60 | 25.2 | intraperitoneal |
| 8 | >50 | 14.9 | intravenous |
| 11 | 30 | 11.6 | intraperitoneal |
| 16 | 37.5 | 8.4 | intravenous |
| 17 | 50 | 6.1 | intravenous |
| 18 | 56.6 | 20.2 | intravenous |
| 20 | 22.5 | 12.6 | intravenous |

The compounds obtained by the present invention are useful as anti-tumor agents. The compounds can be used as they are, or in various forms for administration. For example, when Compounds (I) are used in the form of an injection, they are dissolved in a diluent which is conventionally used in the art such as physiological saline solution or glucose, lactose or mannitol solution for injection. Alternatively, the compounds may be freeze-dried according to the Japanese Pharmacopoeia or may be prepared into injectable powder by adding sodium chloride thereto. In addition, the injection may also contain an auxiliary agent such as polyethylene glycol or HCO-60 (surfactant, manufactured by Nikko Chemical Co., Ltd.), ethanol and/or a carrier such as liposome or cyclodextrin. These injections are generally intravenously administered, but may also be administered intra-arterially, intraperitoneally or intrathoracically.

Compounds (I) may also be formed into tablets, granules, powder or syrup for oral administration with an appropriate excipient, disintegrator, binder or lubricant in a conventional manner. Further, Compounds (I) may be mixed with a conventionally used carrier and formed into suppositories for rectal administration in a conventional manner.

The invention thus also extends to pharmaceutical preparations, both anti-tumor and antibacterial, comprising as the active ingredient a novel mitomycin as defined in admixture with a pharmaceutically acceptable carrier or diluent.

Dosage may appropriately vary according to the administration schedule, the kind of Compounds (I), and the age and condition of a patient. Administration schedule may also be varied according to the condition of a patient and the dosage. For example, the compounds can be intermittently administered in a dose of 0.06 to 6 mg/kg once a week or once every three weeks.

Certain embodiments of the invention are illustrated in the following examples.

Physicochemical data of each compound were obtained by using the following devices:
- NMR:: Bruker AM-400(400 MHz),
Nippon Denshi JNM-GX 270(270 MHz),
Nippon Denshi PS-100 (100 MHz), or
VARIAN EM 390(90 MHz).
- MS:: Hitachi M-80B (measured by the SI method), or
Nippon Denshi JMS-D300 (measured by the FAB method).
- IR:: Nippon Bunko IR-810 (measured by the KBr method).
- TLC:: silica gel Art 5719 or 5715 (manufactured by Merck Inc.).

### Example 1

### 6-Demethyl-6-methoxymethylporfiromycin (Compound 1):

7-Demethoxy-6,7-dihydro-7-ethylenedioxy-6-phenylselenomitomycin F [Compound (VI): R¹ = CH₂OCONH₂, R² = H, Y = Z = CH₃, n = 2] (70 mg) was dissolved in 2.5 ml of chloroform, and 0.2 ml of pyridine and 40 mg of m-chloroperbenzoic acid (purity 70%; hereinafter referred to as MCPBA) were added to the solution at 0°C. After stirring for 5 minutes, 0.5 ml of methanol was added to the mixture. The resulting mixture was stirred at room temperature for 8 hours. Then, 0.5 ml of 6.8 M ammonia/methanol solution was added thereto, followed by stirring at room temperature for 14 hours. The reaction mixture was concentrated and the residue was subjected to silica gel column chromatography. Elution was carried out with chloroform:methanol (96:4, v/v), and the reddish purple fractions were collected. After the solvent was distilled off under reduced pressure, the residue was further purified by TLC, dissolved in chloroform and then precipitated with n-hexane to give 29 mg (yield 59%) of Compound 1 as gray powder.
TLC: R_{f} = 0.49 (CHCl₃:CH₃OH = 9:1)
MS (m/z): 379 (M⁺ +1); C₁₇H₂₂N₄O₆ = 378
NMR (400 MHz, pyridine-d₅) δ: 8.02 (bs, 2H), 7.70 (b, 2H), 5.33 (dd, J=10.6, 4.4Hz, 1H), 4.79 (dd, J=10.6, 11.6Hz, 1H), 4.61 (d, J=11.3Hz, 1H), 4.57 (d, J=11.6Hz, 1H), 4.49 (d, J=12.8Hz, 1H), 3.98 (dd, J=11.6, 4.4Hz, 1H), 3.52 (dd, J=13.0, 2.2Hz, 1H), 3.21 (s, 3H), 3.17 (s, 3H), 2.53 (d, J=4.7Hz, 1H), 2.24 (s, 3H), 2.15 (dd, J=4.7, 2.0Hz, 1H)

### Example 2

### 6-Demethyl-6-methoxymethylmitomycin C (Compound 2):

1a-Acetyl-7-demethoxy-6,7-dihydro-7-ethylenedioxy-6-phenylselenomitomycin A [Compound (VI): R¹ = CH₂OCONH₂, R² = H, Y = CH₃, Z = COCH₃, n = 2] (297 mg) was dissolved in 10 ml of chloroform and 1.0 ml of pyridine, and 200 mg of MCPBA was added to the solution at 0°C. After stirring at room temperature for 10 minutes, 5.0 ml of methanol was added to the mixture. The resulting mixture was stirred at room temperature for 5 hours. The reaction mixture was poured into an aqueous sodium bicarbonate solution and the mixture was extracted with chloroform. The chloroform layer was washed with saturated aqueous sodium chloride solution and then dried over anhydrous sodium sulfate. After the solvent was distilled off under reduced pressure, the residue was subjected to silica gel column chromatography. Elution was carried out with chloroform : methanol (97 : 3, v/v), and yellow fractions were collected. The solvent was distilled off under reduced pressure and the residue was dissolved in 1.0 ml of methanol. Then, 0.5 ml of 6.8 M ammonia/methanol solution was added to the solution, followed by stirring at room temperature for 4 hours. After the solvent was distilled off under reduced pressure, the residue was subjected to silica gel column chromatography and eluted with chloroform : methanol (95 : 5, v/v). Purple fractions were collected, and the solvent was distilled off under reduced pressure to give 53 mg (yield 28%) of Compound 2.
TLC : R_{f}=0.31 (CHCl₃ :CH₃OH = 9 : 1)
MS (m/z): 365(M⁺ +1); C₁₆H₂₀N₄O₆ = 364
IR (cm⁻¹): 3430, 3325, 2922, 1720, 1597, 1543, 1449, 1399, 1338, 1217, 1057, 925, 854, 811, 763, 705
NMR (400MHz, CDCl₃) δ: 6.16(bs, 2H), 4.82(bs, 2H), 4.71(dd, J=10.8, 4.4Hz, 1H), 4.54(t, J=10.6Hz, 1H), 4.44(d, J=12.6Hz, 1H), 4.37(d, J=12.6Hz, 1H), 4.27(d, J=12.8Hz. 1H), 3.64(dd, J=10.4, 4.4Hz, 1H), 3.52(dd, J=12.8, 2.0Hz, 1H), 3.31 (s, 3H), 3.22(s, 3H), 2.90(d, J=4.4Hz, 1H), 2.83(dd, J=4.4, 2.0Hz, 1H), 1.25(bs, 1H)

### Example 3

### 6-Demethyl-6-methoxymethylmitomycin D (Compound 3):

7-Demethoxy-6,7-dihydro-7-ethylenedioxy-6-phenylselenomitomycin B [Compound (VI): R¹=Y=H, R²=CH₂OCONH₂, Z=CH₃, n=2] (368 mg) was dissolved in 20 ml of chloroform and 1.5 ml of pyridine, and 220 mg of MCPBA was added to the solution. After stirring at room temperature for 30 minutes, 5.0 ml of methanol was added to the mixture. The resulting mixture was stirred at room temperature for 15 hours and 0.5 ml of 6.8 M ammonia/methanol solution was added to the mixture. After three hours, the reaction mixture was poured into an aqueous sodium bicarbonate solution. The organic layer was removed, and the water layer was subjected to column chromatography using HP-20 (Mitsubishi Kasei Corporation). Elution was carried out with water : methanol (1 : 1, v/v), and purple fractions were collected. The fractions were concentrated and the residue was subjected to silica gel column chromatography and eluted with chloroform : methanol (95 : 5, v/v). Bluish purple fractions were collected, and the solvent was distilled off under reduced pressure to give 50 mg (yield 20% of Compound 3.
TLC : R_{f} = 0.24 (CHCl₃ : CH₃OH = 9 : 1)
MS (m/z): 367(M⁺ +3), 366(M⁺ +2), 365(M⁺ +1); C₁₆H₂₀N₄O₆ = 364
IR (cm⁻¹): 3400, 1705, 1597, 1542, 1445, 1345, 1158, 1070, 933, 846, 817, 707,
NMR (400MHz, pyridine-d₅) δ: 8.27(bs, 1H), 7.79(bs, 2H), 7.36(bs, 2H), 5.47(dd, J=10.6, 3.7Hz, 1H), 5.19(t, J=10.3Hz, 1H), 4.54(d, J=13.0Hz, 1H), 4.50(d, J=13.0Hz, 1H), 4.42(d, J=13.0Hz, 1H), 4.23(dd, J=10.1, 3.4Hz, 1H), 3.66(dd, J=13.0, 2.0Hz, 1H), 3.16(s, 3H), 2.45(d, J=4.9Hz, 1H), 2.22(dd, J=4.9, 2.0Hz, 1H), 2.12(s, 3H)

### Example 4

### 6-Demethyl-6-methoxymethylmitomycin A (Compound 4) and 1a-acetyl-6-demethyl-6-methoxymethylmitomycin A (Compound 5):

1a-Acetyl-7-demethoxy-6,7-dihydro-7-ethylenedioxy-6-phenylselenomitomycin A (312 mg) was dissolved in 4 ml of chloroform and 0.5 ml of pyridine, and 274 mg of MCPBA was added to the solution at 0°C. After stirring for one hour, 2 ml of methanol was added to the mixture. The resulting mixture was stirred at room temperature for 3 hours. The reaction mixture was poured into an aqueous sodium bicarbonate solution and the mixture was extracted with chloroform. The chloroform layer was washed with saturated aqueous sodium chloride solution and then dried over anhydrous sodium sulfate. The solvent was distilled off under reduced pressure and the residue was subjected to silica gel column chromatography. Elution was carried out with chloroform : methanol (97 : 3, v/v), and yellow fractions were collected. After the solvent was distilled off under reduced pressure, the residue was dissolved in 4 ml of methanol and 1 mg of potassium hydroxide was added to the solution. The mixture was stirred at room temperature for 12 hours and then neutralized with dry ice, followed by addition of saturated aqueous sodium chloride solution. The resulting mixture was extracted with chloroform, and the chloroform layer was dried over anhydrous sodium sulfate and concentrated. The residue was subjected to silica gel column chromatography and eluted with chloroform : methanol (95 : 5, v/v). Reddish purple fractions were collected and treated in a similar manner as in Example 1 to give 21 mg (yield 10%) of powdered Compound 4 and 24 mg (yield 11%) of powdered Compound 5.

### Compound 4:

TLC : R_{f} = 0.47 (CHCl₃ : CH₃OH = 9 : 1)
MS (m/z): 382(M⁺ +3); C₁₇H₂₁N₃O₇ = 379
IR (cm⁻¹): 3450, 2930, 1701, 1640, 1565, 1444, 1400, 1324, 1285, 1215, 1064, 990, 855, 801
NMR (400MHz, CDCl₃) δ: 4.74(b, 2H), 4.74(dd, J=10.8, 4.4Hz, 1H) 4.54(t, J=10.6Hz, 1H), 4.26(d, J=10.1 Hz, 1H), 4.21(d, J=10.1Hz, 1H), 4.18(s, 3H), 4.11 (d, J=12.8Hz, 1H), 3.63(dd, J=10.3, 4.4Hz, 1H), 3.48(dd, J=12.8, 2.0Hz, 1H), 3.37(s, 3H), 3.21 (s, 3H), 2.90(d, J=4.4Hz, 1H), 2.82(dd, J=4.4, 2.0Hz, 1H), 1.26(b, 1H)

### Compound 5:

NMR (100MHz, CDCl₃) δ: major peaks, 4.90(b, 2H), 4.12 (s, 3H), 3.35(s, 3H), 3.17(s, 3H), 2.12(s, 3H)

### Example 5

### 6-Demethyl-6-n-butoxymethylmitomycin C (Compound 6):

Compound 6 (65 mg, yield 23%) was obtained from 400 mg of 1a-acetyl-7-demethoxy-6,7-dihydro-7-ethylenedioxy-6-phenylselenomitomycin A, 257 mg of MCPBA and 3.0 ml of n-butanol in a similar manner as in Example 2.
TLC : R_{f} = 0.43 (CHCl₃ : CH₃OH = 9 : 1)
MS (m/z): 409(M⁺ +3), 408(M⁺ +2), 407(M⁺ +1); C₁₉H₂₆N₄O₆ = 406
IR (cm⁻¹): 3430, 3340, 1717, 1638, 1596, 1559, 1449, 1378, 1335, 1215, 1057, 854, 825, 703
NMR (400MHz, CDCl₃) δ: 6.16(bs, 2H), 4.72(bs, 2H), 4.70(dd, J=10.8, 4.4Hz, 1H) 4.54(b, 1H), 4.47 (d, J=12.6Hz, 1H), 4.40(d, J=12.6Hz, 1H), 4.26 (d, J=12.8Hz, 1H), 3.63(dd, J=10.8, 4.4Hz, 1H), 3.50(bd, J=12.8Hz, 1H), 3.41(t, J=6.6Hz, 2H), 3.21(s, 3H), 2.89(b, 1H), 2.82(b,1H), 1.58-1.30 (m, 4H), 0.91(t, J=7.6Hz, 3H), 0.69(b, 1H)

### Example 6

### 6-Demethyl-6-benzyloxymethylmitomycin C (Compound 7):

Compound 7 (32 mg, yield 27%) was obtained from 150 mg of 1a-acetyl-7-demethoxy-6,7-dihydro-7-ethylenedioxy-6-phenylselenomitomycin A, 96 mg of MCPBA and 0.5 ml of benzyl alcohol in a similar manner as in Example 2.
TLC : R_{f} = 0.34 (CHCl₃ : CH₃OH = 9 : 1)
MS (m/z): 443(M⁺ +3), 442(M⁺ +2), 441(M⁺ +1); C₂₂H₂₄N₄O₆ = 440
IR (cm⁻¹): 3415, 3340, 2916, 1708, 1642, 1595, 1551, 1452, 1404, 1375, 1337, 1207, 1056, 920, 854, 804, 740, 702
NMR (400MHz, CDCl₃) δ: 7.37-7.29(m, 5H), 6.09(bs, 2H), 4.71(dd, J=10.6, 4.4Hz, 1H), 4.65(bs, 2H), 4.55 (bt, J=10.6Hz, 1H), 4.55(d, J=12.6Hz, 1H), 4.49 (d, J=12.6Hz, 1H), 4.46(s, 2H), 4.27(d, J=13.0Hz, 1H), 3.64(dd, J=10.6, 4.4Hz, 1H), 3.52(bd, J=12.3 Hz, 1H), 3.22(s, 3H), 2.90(d, J=4.2Hz, 1H), 2.82(bd, J=4.2Hz, 1H), 0.89(b, 1H)

### Example 7

### 6-Demethyl-6-isopropoxymethylmitomycin C (Compound 8):

1a-Acetyl-7-demethoxy-6-demethyl-6,7-dihydro-7-ethylenedioxy-6-methylenemitomycin A [Compound (II): R¹= CH₂OCONH₂, R²=H, Y=CH₃, Z=COCH₃, n=2] (90 mg) was dissolved in 4 ml of isopropyl alcohol and 3 ml of chloroform, and 0.05 ml of Triton B (1% aqueous solution) was added to the solution. After stirring at room temperature for 2 days, the reaction mixture was neutralized with phosphate buffer, pH 4, and extracted with chloroform. The chloroform layer was washed with saturated aqueous sodium chloride solution and then dried over anhydrous sodium sulfate. After the solvent was distilled off under reduced pressure, the residue was subjected to silica gel column chromatography. Elution was carried out with chloroform : methanol (97 : 3, v/v), and yellow fractions were collected. The solvent was distilled off under reduced pressure to give 53 mg (yield 52%) of 1a-acetyl-6-demethyl-6,7-dihydro-7-ethylenedioxy-6-isopropoxymethylmitomycin A [Compound (III):
R¹=CH₂OCONH₂, R²=H, W=OCH(CH₃)₂, Y=CH₃, Z=COCH₃, n=2].
NMR (90MHz, CDCl₃) δ: major peaks, 4.95 (b, 2H), 3.20, 3.15(s, 3H), 2.12, 2.09(s, 3H), 1.00, 0.98(d, J=6Hz, 6H)
The above compound was dissolved in 3 ml of methanol, and 0.3 ml of 6.8 M ammonia/methanol solution was added to the solution, followed by stirring at 0°C for 16 hours. The solvent was distilled off under reduced pressure and the residue was subjected to silica gel column chromatography. Elution was carried out with chloroform : methanol (95 : 5, v/v), and purple fractions were collected. After the solvent was distilled off under reduced pressure, the residue was dissolved in a small quantity of chloroform, and n-hexane was added to the solution to give powder. Then, the solvent was distilled off under reduced pressure. The residue was thoroughly dried in vacuo at room temperature to give 26 mg (yield 60%) of Compound 8 as gray powder.
TLC : R_{f}=0.36 (CHCl₃ : CH₃OH = 9 : 1)
MS (m/z): 394(M⁺ +2); C₁₈H₂₄N₄O₆ = 392
IR (cm⁻¹): 3288, 2968, 1721, 1647, 1592, 1561, 1441, 1404, 1369, 1265, 1218, 1166, 1121, 950, 924, 853, 822, 763, 702
NMR (400MHz, CDCl₃) δ: 6.19(b, 2H), 4.78(b, 2H), 4.70(dd, J=10.6, 4.4Hz, 1H), 4.51(bt, 1H), 4.49 (d, J=12.6Hz, 1H), 4.40(d, J=12.3Hz, 1H), 4.26 (d, J=13.0Hz, 1H), 3.65-3.59(m, 1H), 3.62(dd, J=10.6, 4.4Hz, 1H), 3.51(bd, J=13.0Hz, 1H), 3.21 (s, 3H), 2.90(b, 1H), 2.82(b, 1H), 1.18(d, J=5.9 Hz, 3H), 1.17(d, J=6.2Hz, 3H), 0.64(b, 1H)

### Example 8

### 6-Demethyl-6-(3,4-dimethoxybenzyloxy)methylmitomycin C (Compound 9):

Compound 9 (30 mg, yield 8%) was obtained from 435 mg of 1a-acetyl-7-demethoxy-6,7-dihydro-7-ethylenedioxy-6-phenylselenomitomycin A, 200 mg of MCPBA and 1.0 ml of 3,4-dimethoxybenzyl alcohol in a similar manner as in Example 2.
TLC : R_{f}=0.41 (CHCl₃ :CH₃OH = 9 : 1)
MS (m/z): 503(M⁺+3), 502(M⁺+2), 501(M⁺+1); C₂₉H₂₈N₄O₈=500
IR (cm⁻¹): 3440, 3330, 2925, 1708, 1647, 1597, 1551, 1512, 1455, 1337, 1262, 1136, 1054, 920, 852, 806, 755, 701
NMR (400MHz, CDCl₃) δ: 6.89(d, J=8.9Hz, 1H), 6.86(s, 1H), 6.83(d, J=8.6Hz, 1H), 6.13(b, 2H), 4.85(b, 2H), 4.70(dd, J=10.6, 4.4Hz, 1H), 4.52(d, J=12.6 Hz, 1H), 4.51(t, J=10.6Hz, 1H), 4.46(d, J=12.6Hz, 1H), 4.39(s, 2H), 4.26(d, J=12.8Hz, 1H), 3.88(s, 3H), 3.87(s, 3H), 3.63(dd, J=10.6, 4.4Hz, 1H), 3.52(dd, J=12.8, 1.7Hz, 1H), 3.21(s, 3H), 2.90(d, J=4.4Hz, 1H), 2.82(dd, J=4.2, 1.7Hz, 1H)

### Example 9

### 6-Demethyl-6-hydroxymethylmitomycin C (Compound 10):

1a-Acetyl-7-demethoxy-6-demethyl-6,7-dihydro-7-ethylenedioxy-6-(3,4-dimethoxybenzyloxy)methylmitomycin A [Compound (III): R¹=CH₂OCONH₂, R²=H, 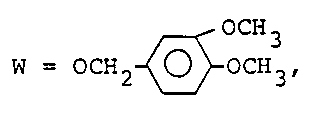
Y=CH₃, Z=COCH₃, n=2] (110 mg, yield 38%) was obtained from 207 mg of 1a-acetyl-7-demethoxy-6-demethyl-6,7-dihydro-7-ethylenedioxy-6-methylenemitomycin A, 2.0 ml of 3,4-dimethoxybenzyl alcohol, 0.05 ml of Triton B and 15 ml of chloroform in a similar manner as in Example 7.

The obtained compound (34 mg) was dissolved in 3.0 ml of chloroform, and 0.3 ml of water and 15.8 mg of DDQ were added to the solution at 0°C, followed by stirring at room temperature for 6 hours. After insoluble matters were filtered off, the filtrate was concentrated and the residue was purified by preparative TLC (chloroform : methanol = 9 : 1) to give 10 mg (yield 40%) of 1a-acetyl-7-demethoxy-6-demethyl-6,7-dihydro-7-ethylenedioxy-6-hydroxymethylmitomycin A [Compound (III): R¹=CH₂OCONH₂, R²=H, W=OH, Y=CH₃, Z= COCH₃, n=2].

Compound 10 (1.2 mg, yield 60%) was obtained from 2.5 mg of the thus obtained compound, 2.0 ml of methanol and 0.3 ml of 6.8 M ammonia/methanol solution in a similar manner as in Example 7.
TLC : R_{f}=0.24 (CHCl₃ : CH₃OH = 85 : 15)
MS (m/z): 351(M⁺+1); C₁₅H₁₈N₄O₆ = 350
IR (cm⁻¹): 3420, 2930, 1709, 1655, 1600, 1562, 1541, 1450, 1336, 1220, 1063, 973, 857, 825, 764, 700
NMR (400MHz, pyridine-d₅) δ: 7.94(b, 2H), 7.62(b, 2H), 5.39(dd, J=10.3, 4.2Hz, 1H), 5.06(bt, J=11.1Hz, 1H), 5.05(d, J=12.5Hz, 1H), 5.00(d, J=12.3Hz, 1H), 4.50(d, J=12.8Hz, 1H), 4.01(dd, J=11.1, 4.2Hz, 1H), 3.57(dd, J=12.8, 2.0Hz, 1H), 3.18(s, 3H), 3.13(d, J=4.4Hz, 1H), 2.72(dd, J=4.4, 2.0Hz, 1H), 1.29(b, 1H)

### Example 10

### 6-Demethyl-6-ethylthiomethylmitomycin C (Compound 11):

Compound 11 (39 mg, yield 29%) was obtained from 200 mg of 1a-acetyl-7-demethoxy-6,7-dihydro-7-ethylenedioxy-6-phenylselenomitomycin A, 128 mg of MCPBA and 0.05 ml of ethanethiol in a similar manner as in Example 2.
TLC : R_{f}=0.39 (CHCl₃ :CH₃OH=9 :1)
MS (m/z): 395 (M⁺+1); C₁₇H₂₂N₄O₅S = 394
IR (cm⁻¹): 3430, 3330, 2920, 1705, 1662, 1600, 1550, 1445, 1338, 1220, 1068, 956, 850, 760, 700
NMR (400MHz, CDCl₃) δ: 5.97(bs, 2H), 4.76(bs, 2H), 4.70(dd, J=10.8, 4.4Hz, 1H), 4.53(bt, J=10.6Hz, 1H), 4.25(d, J=12.8Hz, 1H), 3.66(d, J=13.8Hz, 1H), 3.63(dd, J=10.1, 4.4Hz, 1H), 3.52(d, J=12.8Hz, 1H), 3.50(d, J=13.3Hz, 1H), 3.22(s, 3H), 2.90(d, J=4.2Hz, 1H), 2.83(bs, 1H), 2.50(q, J=7.4Hz, 2H), 1.27(t, J=7.4Hz, 3H), 0.89(b, 1H)

### Example 11

### 6-Demethyl-6-(2-methoxycarbonylethylthio)methylmitomycin C (Compound 12):

Compound 12 (66 mg, yield 24%) was obtained from 350 mg of 1a-acetyl-7-demethoxy-6,7-dihydro-7-ethylenedioxy-6-phenylselenomitomycin A, 10 ml of chloroform, 0.5 ml of pyridine, 155 mg of MCPBA and 0.1 ml of methyl 3-mercaptopropionate in a similar manner as in Example 2.
TLC : R_{f} = 0.29 (CHCl₃ : CH₃OH = 9 : 1)
MS (m/z): 454(M⁺ + 2); C₁₉H₂₄N₄O₇S = 452
IR (cm⁻¹): 3415, 2920, 1709, 1597, 1560, 1435, 1339, 1220, 1069, 850, 805, 758, 700
NMR (400MHz, CDCl₃) δ: 6.15(b, 2H), 4.98(b, 2H), 4.69 (dd, J=10.8, 4.4Hz, 1H), 4.49(bt, J=10.3Hz,1H), 4.25(d, J=13.0Hz, 1H), 3.70(s, 3H), 3.65 (d, J=13.5 Hz, 1H), 3.62(dd, J=10.3, 4.7Hz, 1H), 3.54(d, J=13.0Hz, 1H), 3.52(bd, J=12.8Hz, 1H), 3.23(s, 3H), 2.91(bs, 1H), 2.83(bs, 1H), 2.76-2.66(m, 4H), 0.73(b, 1H)

### Example 12

### 6-Demethyl-6-(2-hydroxyethylthio)methylmitomycin C (Compound 13):

1a-Acetyl-7-demethoxy-6,7-dihydro-7-ethylenedioxy-6-(2-hydroxyethylthio)methylmitomycin A [Compound (III):
R¹=CH₂OCONH₂, R²=H, W=S (CH₂)₂OH, Y=CH₃, Z=COCH₃, n=2] (129 mg, yield 60%) was obtained from 273 mg of 1a-acetyl-7-demethoxy-6,7-dihydro-7-ethylenedioxy-6-phenylselenomitomycin A, 6 ml of tetrahydrofuran, 0.5 ml of pyridine, 151 mg of MCPBA and 104 mg of 2-mercaptoethanol in a similar manner as in Example 2.

NMR (90MHz, CDCl₃)δ: major peaks, 5.24(b, 2H), 3.23, 3.21(s, 3H), 2.05(s, 3H)
Compound 13 (64 mg, yield 55%) was obtained as brown powder from the above compound, 10 ml of methanol and 1.0 ml of 6.8 M ammonia/methanol solution in a similar manner as in Example 7.
TLC : R_{f}=0.29 (CHCl₃ : CH₃OH = 9 : 1)
MS (m/z): 411 (M⁺+1); C₁₇H₂₂N₄O₆S = 410
IR (cm⁻¹): 3450, 2930, 1715, 1655, 1599, 1551, 1434, 1334, 1225, 1065, 860, 806, 760
NMR (400MHz, pyridine-d₅) δ: 8.15(b, 2H), 7.60(b, 2H), 6.67(b, 1H), 5.39(dd, J=10.6, 4.2Hz, 1H), 5.07 (bt, 1H), 4.53(d, J=12.8Hz, 1H), 4.12(t, J=6.2Hz, 2H), 4.08(d, J=12.8Hz, 1H), 4.00(dd, J=11.1, 4.2Hz, 1H), 3.95(d, J=13.0Hz, 1H), 3.58(bd, J=12.6Hz, 1H), 3.17(s, 3H), 3.12(bs, 1H), 2.92-2.87 (m, 2H), 2.73(bs, 1H), 2.07(b, 1H)

### Example 13

### 6-Demethyl-6-dodecylthiomethylmitomycin C (Compound 14):

1a-Acetyl-7-demethoxy-6-demethyl-6,7-dihydro-7-ethylenedioxy-6-n-dodecylthiomethylmitomycin A [Compound (III): R¹=CH₂OCONH₂, R²=H, W=S(CH₂)₁₁CH₃, Y=CH₃, Z=COCH₃, n=2] (200 mg, yield 62%) was obtained from 300 mg of 1a-acetyl-7-demethoxy-6,7-dihydro-7-ethylenedioxy-6-phenylselenomitomycin A, 5.5 ml of chloroform, 0.3 ml of pyridine, 172 mg of MCPBA and 0.7 ml of 1-dodecanethiol in a similar manner as in Example 2.

NMR (100MHz, CDCl₃) δ: major peaks, 5.34(b, 2H), 3.28 (s, 3H), 2.12(s, 3H), 1.6-1.2(m, 20H), 0.86(t, J=6Hz, 3H)
Compound 14 (35 mg, yield 20%) was obtained as reddish purple powder from the above compound, methanol and 1.0 ml of 6.8 M ammonia/methanol solution in a similar manner as in Example 7.
TLC : R_{f}=0.51 (CHCl₃ :CH₃OH=9 : 1)
MS (m/z): 537 (M⁺+3); C₂₇H₄₂N₄O₅S = 534
IR (cm⁻¹): 3440, 3322, 2924, 2854, 1729, 1649, 1598, 1555, 1436, 1333, 1220, 1162, 1070, 992, 953, 926, 853, 812, 783, 760, 703
NMR (400MHz, CDCl₃) δ: 5.97(b, 2H), 4.78(b, 2H), 4.70(dd, J=10.6, 4.4Hz, 1H), 4.53(t, J=10.6Hz, 1H), 4.25(d, J=12.8Hz, 1H), 3.63(d, J=13.5Hz, 1H), 3.63(dd, J=11.1, 4.4Hz, 1H), 3.52(dd, J=12.8, 1.7Hz, 1H), 3.48(d, J=13.3Hz, 1H), 3.22(s, 3H), 2.90(d, J=4.4Hz, 1H), 2.82(dd, J=4.2, 1.5Hz, 1H), 2.47(m, 2H), 1.59(m, 2H), 1.37-1.25(m, 18H), 0.88(t, J=6.9Hz, 3H), 0.70(b, 1H)

### Example 14

### 6-Demethyl-6-(2,3-dihydroxypropylthio)methylmitomycin C (Compound 15):

Compound 15 (38 mg, yield 24%) was obtained from 147 mg of 1a-acetyl-7-demethoxy-6-demethyl-6,7-dihydro-7-ethylenedioxy-6-methylenemitomycin A, 10 ml of chloroform and 50 mg of 3-mercapto-1,2-propanediol in a similar manner as in Example 7. Purification was carried out by preparative TLC (chloroform : methanol = 8 : 2, v/v).

On the basis of NMR spectrum, Compound 15 was found to be a 1 :1 diastereomeric mixture with asymmetric carbon on the side chain at the 6-position.
TLC : R_{f}=0.19 (CHCl₃ : CH₃OH = 8 : 2)
MS (m/z): 441 (M⁺+1); C₁₈H₂₄N₄O₇S = 440
IR (cm⁻¹): 3420, 2930, 1712, 1599, 1541, 1433, 1336, 1218, 1068, 853, 801, 764
NMR (400MHz, pyridine-d₅) δ: 8.17(b, 2H), 7.63(b, 2H), 5.38(dd, J=10.3, 4.2Hz, 1H), 5.04(b, 1H), 4.54(d, J=12.8Hz, 0.5H), 4.33(d, J=12.8Hz, 0.5H), 4.46-4.39(m, 1H), 4.16-4.04(m, 5H), 3.57(bd, J=12.3Hz, 1H), 3.17(s, 1.5H), 3.16(s, 1.5H), 3.14-3.01(m, 2H), 3.11(bs, 1H), 2.72(bs, 1H), 2.07(b, 1H)

### Example 15

### 6-Demethyl-6-isopropylthiomethylmitomycin C (Compound 16):

1a-Acetyl-7-demethoxy-6-demethyl-6,7-dihydro-7-ethylenedioxy-6-isopropylthiomethylmitomycin A [Compound (III): R¹=CH₂OCONH₂, R²=H, W=SCH(CH₃)₂, Y=CH₃, Z= COCH₃, n=2] (231 mg, yield 85%) was obtained from 230 mg of 1a-acetyl-7-demethoxy-6-demethyl-6,7-dihydro-7-ethylenedioxy-6-methylenemitomycin A, 10 ml of chloroform and 50 mg of 2-propanethiol in a similar manner as in Example 7.
NMR (90MHz, CDCl₃) δ: major peaks, 5.17(b, 2H), 3.21, 3.18(s, 3H), 2.10(s, 3H), 1.32-1.17(m, 6H)

Compound 16 (65 mg, yield 34%) was obtained from the above compound, 20 ml of methanol and 0.5 ml of 6.8 M ammonia/methanol solution in a similar manner as in Example 7.
TLC : R_{f}=0.42 (CHCl₃ :CH₃OH = 9 : 1)
MS (m/z): 409 (M⁺ +1); C₁₈H₂₄N₄O₅S = 408
IR (cm⁻¹): 3276, 2954, 1715, 1639, 1595, 1556, 1441, 1332, 1220, 1170, 1060, 953, 853, 820, 764, 700
NMR (400MHz, pyridine-d₅) δ: 8.06(b, 2H), 7.60(b, 2H), 5.40(dd, J=10.6, 4.4Hz, 1H), 5.07(t, J=10.8Hz, 1H), 4.57(d, J=12.8Hz, 1H), 4.02(dd, J=11.3, 4.4 Hz, 1H), 3.98(d, J=13.3Hz, 1H), 3.82(d, J=13.3 Hz, 1H), 3.62(dd, J=12.8, 2.0Hz, 1H), 3.19(s, 3H), 3.13(d, J=4.4Hz, 1H), 3.00-2.93(m, 1H), 2.74 (dd, J=4.4, 2.0Hz, 1H), 1.30(b, 1H), 1.28(d, J=6.6Hz, 3H), 1.25(d, J=6.9Hz, 3H)

### Example 16

### 6-Demethyl-6-phenylthiomethylmitomycin C (Compound 17):

1a-Acetyl-7-demethoxy-6,7-dihydro-7-ethylenedioxy-6-phenylselenomitomycin A (292 mg) was dissolved in 15 ml of chloroform, and 140 mg of potassium carbonate and 149 mg of MCPBA were added to the solution under ice cooling. After stirring at room temperature for 2 hours, the reaction mixture was poured into an aqueous sodium thiosulfate solution. The mixture was extracted with chloroform, and the chloroform layer was washed with saturated aqueous sodium chloride solution and then dried over anhydrous sodium sulfate. After the drying agent was filtered off, 0.06 ml of thiophenol was added to the filtrate, followed by stirring at room temperature for 3.5 hours. The reaction mixture was washed with an aqueous sodium bicarbonate solution and saturated aqueous sodium chloride solution, dried over anhydrous sodium sulfate, and then concentrated. The residue was subjected to silica gel column chromatography. Elution was carried out with chloroform : methanol (97 : 3, v/v), and reddish purple fractions were collected. The solvent was distilled off under reduced pressure to give 142 mg (yield 51%) of 1a-acetyl-7-demethoxy-6-demethyl-6,7-dihydro-7-ethylenedioxy-6-phenylthiomethylmitomycin A [Compound (III): R¹=CH₂OCONH₂, R²=H, 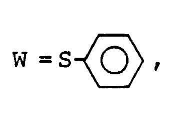
Y=CH₃, Z=COCH₃, n=2].
NMR (90MHz, CDCl₃) δ: major peaks, 7.42-7.20(m, 5H), 5.10(b, 2H), 3.22, 3.20(s, 3H), 2.10, 2.08(s, 3H)
Compound 17 (27 mg, yield 29%) was obtained from 110 mg of the above compound, 25 ml of methanol and 0.3 ml of 6.8 M ammonia/methanol solution in a similar manner as in Example 7.
TLC : R_{f}=0.45 (CHCl₃ : CH₃OH = 9 : 1)
MS (m/z): 443 (M⁺+1); C₂₁H₂₂N₄O₅S = 442
IR (cm⁻¹): 3425, 3320, 2886, 1708, 1650, 1600, 1553, 1480, 1439, 1334, 1221, 1165, 958, 852, 765, 740, 690
NMR (400MHz, CDCl₃) δ: 7.40-7.18(m, 5H), 5.85(b, 2H), 4.79(b, 2H), 4.68(dd, J=10.6, 4.4Hz, 1H), 4.51 (bt, 1H), 4.21(d, J=13.0Hz, 1H), 4.00(d, 12.3Hz, 1H), 3.89(d, J=12.6Hz, 1H), 3.60(dd, J=10.6, 4.4 Hz, 1H), 3.50(bd, J=13.7Hz, 1H), 3.19(s, 3H), 2.89(b, 1H), 2.81(b, 1H), 0.64(b, 1H)

### Example 17

### 6-Demethyl-6-benzylthiomethylmitomycin C (Compound 18):

Compound 18 (30 mg, yield 21%) was obtained from 184 mg of 1a-acetyl-7-demethoxy-6,7-dihydro-7-ethylenedioxy-6-phenylselenomitomycin A, 88 mg of potassium carbonate, 14 ml of chloroform, 106 mg of MCPBA and 0.05 ml of benzyl mercaptan in a similar manner as in Example 16.
TLC : R_{f}=0.46 (CHCl₃ : CH₃OH = 9 : 1)
MS (m/z): 458 (M⁺+2); C₂₂H₂₄N₄O₅S = 456
IR (cm⁻¹): 3420, 3320, 2914, 1709, 1655, 1599, 1560, 1475, 1449, 1334, 1220, 1165, 1064, 960, 852, 802, 763, 703
NMR (400MHz, CDCl₃) δ: 7.33-7.21(m, 5H), 5.67(b, 2H), 4.74(b, 2H), 4.67(dd, J=10.8, 4.4Hz, 1H), 4.52 (bt, 1H), 4.24(d, J=13.0Hz, 1H), 3.71(d, J=13.5 Hz, 1H), 3.68(d, J=13.5Hz, 1H), 3.60(dd, J=10.6, 4.4Hz, 1H), 3.52(d, J=13.3Hz, 1H), 3.51(bd, J=13.0Hz, 1H), 3.47(d, J=13.3Hz, 1H), 3.21(s, 3H), 2.88(b, 1H), 2.82(b, 1H), 0.52(b, 1H)

### Example 18

### Compound 19 [Compound (I): R¹=CH₂OCONH₂, R²=H, W-X= S(CH₂)₂NH, Y=CH₃, Z=COCH₃]:

Compound 19 (55 mg, yield 23%) was obtained from 320 mg of 1a-acetyl-7-demethoxy-6,7-dihydro-7-ethylenedioxy-6-phenylselenomitomycin A, 10 ml of tetrahydrofuran, 0.7 ml of pyridine, 179 mg of MCPBA and 127 mg of 2-aminoethanethiol hydrochloride in a similar manner as in Example 2.
NMR (90MHz, CDCl₃) δ: major peaks, 6.80(b, 1H), 5.02 (b, 2H), 3.20(s, 3H), 2.11(s, 3H)

### Example 19

### Compound 20 [Compound (I): R¹=CH₂OCONH₂, R²=Z=H, W-X= S(CH₂)₂NH, Y = CH₃]:

Compound 19 (55 mg) obtained in Example 18 was dissolved in 10 ml of methanol, and 0.5 ml of 6.8 M ammonia/methanol solution was added to the solution. The mixture was stirred at room temperature for 4 hours. The reaction mixture was concentrated under reduced pressure and the residue was purified by preparative TLC (chloroform : methanol = 9 : 1, v/v) to give 33 mg (yield 66%) of Compound 20.
TLC : R_{f}=0.25 (CHCl₃ : CH₃OH = 9 : 1)
MS (m/z: 394 (M⁺+2); C₁₇H₂₀N₄O₅S = 392
IR (cm⁻¹): 3440, 3310, 2930, 1711, 1633, 1600, 1554, 1507, 1452, 1338, 1202, 1152, 1070, 960, 847, 806, 756, 705
NMR (400MHz, CDCl₃) δ: 6.70(b, 1H), 4.82(b, 2H), 4.68 (dd, J=10.6, 4.4Hz, 1H), 4.49(t, J=10.6Hz, 1H), 4.27(d, J=13.0Hz, 1H), 3.91(bt, J=5.4Hz, 2H), 3.87(d, J=16.2Hz, 1H), 3.82(d, J=16.2Hz, 1H), 3.60(dd, J=10.6, 4.4Hz, 1H), 3.51(dd, J=13.0, 1.7 Hz, 1H), 3.20(s, 3H), 3.08(bt, J=5.4Hz, 2H), 2.90 (d, J=4.4Hz, 1H), 2.82(dd, J=4.4, 1.7Hz, 1H), 0.90(b, 1H)

### Example 20

### 6-Demethyl-6-allyloxymethylmitomycin C (Compound 21):

1a-Acetyl-7-demethoxy-6-demethyl-6,7-dihydro-7-ethylenedioxy-6-allyloxymethylmitomycin A [Compound (III): R¹=CH₂OCONH₂, R²=H, W=OCH₂CH=CH₂, Y=CH₃, Z=COCH₃, n= 2] (49.2 mg, yield 42%) was obtained from 102 mg of 1a-acetyl-7-demethoxy-6-demethyl-6,7-dihydro-7-ethylenedioxy-6-methylenemitomycin A, 5.0 ml of allyl alcohol and 5.0 µl of Triton B (40% aqueous solution) in a similar manner as in Example 7.

Compound 21 (16.4 mg, yield 41%) was obtained as purple powder from 49.2 mg of the above compound, 5.0 ml of methanol and 0.50 ml of 6.1 M ammonia/methanol solution in a similar manner as in Example 7.
TLC : R_{f}=0.39 (CHCl₃ :CH₃OH=9 : 1)
MS (m/z): 391 (M⁺+1); C₁₈H₂₂N₄O₆ = 390
IR (cm⁻¹): 3430, 3320, 3200, 2930, 1710, 1600, 1550, 1450, 1400, 1340, 1210, 1060
NMR (270MHz, pyridine-d₅) δ: 8.3-7.9(br, 2H), 7.9-7.4 (br, 2H), 5.95(m, 1H), 5.42(dd, J=4.2, 10.3Hz, 1H), 5.30(ddd, J=17.4, 3.7, 1.8Hz, 1H), 5.2-5.0 (m, 1H), 5.09(ddd, J=10.4, 3.3, 1.5Hz, 1H), 4.71 (d, J=11.7Hz, 1H), 4.64(d, J=12.6Hz, 1H), 4.57(d, J=12.6Hz, 1H), 4.04(dd, J=11.2, 4.2Hz, 1H), 3.97 (m, 2H), 3.60(d, J=12.8Hz, 1H), 3.20(s, 3H), 3.18 (bs, 1H), 2.75(bs, 1H), 2.15(bs, 1H)

### Example 21

### 6-Demethyl-6-(2-methoxyethoxy)methylmitomycin C (Compound 22):

1a-Acetyl-7-demethoxy-6-demethyl-6,7-dihydro-7-ethylenedioxy-6-(2-methoxyethoxy)methylmitomycin A [Compound (III): R¹=CH₂OCONH₂, R²=H, W=OCH₂CH₂OCH₃, Y=CH₃, Z=COCH₃, n=2] (61.8 mg, yield 17%) was obtained from 210 mg of 1a-acetyl-7-demethoxy-6-demethyl-6,7-dihydro-7-ethylenedioxy-6-methylenemitomycin A, 30 ml of 2-methoxyethanol and 10 µl of Triton B (40% aqueous solution) in a similar manner as in Example 7.

The obtained compound (61.8 mg) was dissolved in 20 ml of anhydrous tetrahydrofuran. The solution was allowed to stand at room temperature for 7 days in an ammonia gas atmosphere. After the solvent was distilled off under reduced pressure, the residue was subjected to post-treatment and purification in a similar manner as in Example 7 to give 4.3 mg (yield 8.4%) of Compound 22 as purple powder.
TLC : R_{f}=0.25 (CHCl₃ :CH₃OH=9 : 1)
MS (m/z): 409 (M⁺ +1); C₁₈H₂₄N₄O₇ = 408
IR (cm⁻¹): 3410, 3300, 3200, 2920, 1710, 1600, 1550, 1450, 1340, 1070
NMR (90MHz, pyridine-d₅) δ: major peaks, 8.1-7.0(br, 4H), 5.32(dd, J=11, 4Hz, 1H), 4.97(t, J=11Hz, 1H), 4.64(bs, 2H), 4.50(d, J=13Hz, 1H), 3.94(dd, J=11, 4Hz, 1H), 3.7-3.3(m, 5H), 3.24(s, 3H), 3.19 (s, 3H)

### Example 22

### 6-Demethyl-6-[(4-hydroxyphenyl)thio]methylmitomycin C (Compound 23):

1A-Acetyl-7-demethoxy-6-demethyl-6,7-dihydro-7-ethylenedioxy-6-[(4-hydroxyphenyl)thio]methylmitomycin A [Compound (III): R¹=CH₂OCONH₂, R²=H, 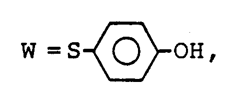
Y=CH₃, Z=COCH₃, n=2] (190 mg, yield 68%) was obtained from 297 mg of 1a-acetyl-7-demethoxy-6,7-dihydro-6-phenylselenomitomycin A, 161 mg of MCPBA, 25 ml of chloroform and 120 µl of 4-hydroxythiophenol in a similar manner as in Example 2.

The obtained compound (154 mg) was dissolved in 29 ml of methanol, and 400 mg of ammonium acetate was added to the solution. The mixture was stirred at room temperature for 9 hours and then diluted with chloroform. The diluted mixture was washed with saturated aqueous sodium chloride solution three times and dried over anhydrous sodium sulfate. After the solvent was distilled off under reduced pressure, the residue was subjected to purification and drying in a similar manner as in Example 7 to give 37.8 mg (yield 29%) of Compound 23 as grayish green powder.
TLC : R_{f} = 0.32 (CHCl₃ : CH₃OH=9 :1)
MS (m/z): 460 (M⁺ +2); C₂₁H₂₂N₄O₅S = 458
IR (cm⁻¹): 3450, 3370, 2950, 1710, 1660, 1580, 1500, 1340, 1270, 1080, 1040
NMR (270MHz, pyridine-d₅) δ: 12-11(br, 1H), 8.3-8.0 (br, 2H), 7.9-7.3(br, 2H), 7.6-7.5(m, 2H), 7.1-7.0(m, 2H), 5.42(dd, J=10.4, 4.3Hz, 1H), 5.11(bt, J=11.2Hz, 1H), 4.49(d, J=12.7Hz, 1H), 4.35(d, J=12.2Hz, 1H), 4.25(d, J=12.0Hz, 1H), 4.02(dd, J=11.2, 4.2Hz, 1H), 3.54(bd, J=13.4Hz, 1H), 3.17(s, 3H), 3.13(bs, 1H), 2.71(bs, 1H), 2.09(bt, J=7.1 Hz, 1H)

### Example 23

### 6-Demethyl-6-[(4-chlorophenyl)thio]methylmitomycin C (Compound 24):

1a-Acetyl-7-demethoxy-6-demethyl-6,7-dihydro-7-ethylenedioxy-6-[(4-chlorophenyl)thio]methylmitomycin A [Compound (III): R¹=CH₂OCONH₂, R²=H, 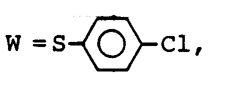
Y= CH₃, Z=COCH₃, n=2] (184 mg, yield 62%) was obtained from 219 mg of 1a-acetyl-7-demethoxy-6-demethyl-6,7-dihydro-7-ethylenedioxy-6-methylenemitomycin A, 153 mg of 4-chlorothiophenol, 0.15 ml of triethylamine and 50 ml of chloroform in a similar manner as in Example 7.

Compound 24 (34.3 mg, yield 36%) was obtained as purple powder from 114 mg of the above compound, 30 ml of methanol and 1.1 ml of 6.1 M ammonia/methanol solution in a similar manner as in Example 7.
TLC : R_{f}=0.31 (CHCl₃ :CH₃OH = 9 : 1)
MS (m/z): 477, 479 (M⁺+1); C₂₁H₂₁ClN₄O₅S = 476.5
IR (cm⁻¹): 3420, 3320, 3220, 2930, 1710, 1600, 1550, 1470, 1450, 1340, 1220, 1070
NMR (90MHz, pyridine-d₅) δ: 8.4-7.8(br, 4H), 7.6-7.0 (m, 4H), 5.27(dd, J=10, 4Hz, 1H), 4.93(t, J=11Hz, 1H), 4.40(d, J=13Hz, 1H), 4.22(bs, 2H), 3.91(dd, J=11, 5Hz, 1H), 3.49(d, J=13Hz, 1H), 3.19(s, 3H), 3.05(bs, 1H), 2.70(bs, 1H), 1.8(bs, 1H)

### Example 24

### 6-Demethyl-6-[(4-methoxyphenyl)thio]methylmitomycin C (Compound 25):

1a-Acetyl-7-demethoxy-6-demethyl-6,7-dihydro-7-ethylenedioxy-6-[(4-methoxyphenyl)thio]methylmitomycin A [Compound (III): R=CH₂OCONH₂, R²=H, 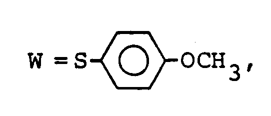
Y=CH₃, Z=COCH₃, n=2] (186 mg, yield 63%) was obtained from 219 mg of 1a-acetyl-7-demethoxy-6-demethyl-6,7-dihydro-7-ethylenedioxy-6-methylenemitomycin A, 134 mg of 4-methoxythiophenol, 0.15 ml of triethylamine and 50 ml of chloroform in a similar manner as in Example 7.

Compound 25 (47.4 mg, yield 37%) was obtained as purple powder from 154 mg of the above compound, 15 ml of methanol and 1.0 ml of 6.1 M ammonia/methanol solution in a similar manner as in Example 7.
TLC : R_{f}=0.49 (CHCl₃ : CH₃OH = 9 :1)
MS (m/z): 473 (M⁺+1); C₂₂H₂₄N₄O₆S = 472
IR (cm⁻¹): 3420, 3310, 3200, 2920, 1710, 1590, 1490, 1440, 1330, 1240, 1070
NMR (270MHz, pyridine-d₅) δ: 8.4-8.1(br, 2H), 7.9-7.4 (br, 2H), 7.6-7.5(m, 2H), 6.9-6.8(m, 2H), 5.42 (dd, J=10.4, 4.2Hz, 1H), 5.09(bt, J=10.2Hz, 1H), 4.45(d, J=12.6Hz, 1H), 4.32(d, J=12.1Hz, 1H), 4.26(d, J=11.9Hz, 1H), 4.02(dd, J=11.2, 4.2Hz), 1H), 3.61(s, 3H), 3.55(d, J=12.1Hz, 1H), 3.18(s, 3H), 3.12(bs, 1H), 2.72(bs, 1H), 2.12(br, 1H)

### Example 25

### 6-Demethyl-6-[(4-nitrophenyl)thio]methylmitomycin C (Compound 26):

1a-Acetyl-7-demethoxy-6-demethyl-6,7-dihydro-7-ethylenedioxy-6-[(4-nitrophenyl)thio]methylmitomycin A [Compound (III): R¹=CH₂OCONH₂, R²=H, 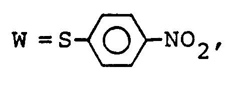
Y=CH₃, Z=COCH₃, n=2] (289 mg, yield 68%) was obtained from 311 mg of 1a-acetyl-7-demethoxy-6-demethyl-6,7-dihydro-7-ethylenedioxy-6-methylenemitomycin A, 145 mg of 4-nitrothiophenol (purity 80%), 0.10 ml of triethylamine and 30 ml of dichloromethane in a similar manner as in Example 7.

Compound 26 (7.7 mg, yield 4%) was obtained as gray powder from 225 mg of the above compound, 20 ml of anhydrous tetrahydrofuran and ammonia gas in a similar manner as in Example 21.
TLC : R_{f}=0.34 (CHCl₃ :CH₃OH = 9 :1)
MS (m/z): 488 (M⁺+1); C₂₁H₂₁N₅O₇S = 487
IR (cm⁻¹): 3420, 3320, 3200, 2920, 1710, 1590, 1550, 1500, 1470, 1440, 1330, 1220, 1070
NMR (270MHz, pyridine-d₅) δ: 8.03(dd, J=7.1, 1.9Hz, 2H), 7.9-7.4(br, 4H), 7.33(dd, J=7.1, 1.9Hz, 2H), 5.44(dd, J=10.3, 4.2Hz, 1H), 5.11(bt, J=12Hz, 1H), 4.58(d, J=12.8Hz, 1H), 4.43(d, J=11.5Hz, 1H), 4.34(d, J=11.5Hz, 1H), 4.06(dd, J=11.1, 4.1Hz, 1H), 3.62(d, J=11.4Hz, 1H), 3.21(s, 3H), 3.15(bs, 1H), 2.77(bs, 1H), 2.17(br, 1H)

### Example 26

### Compound 27 [Compound (I): R¹=CH₂OCONH₂, R²=H, W-X=

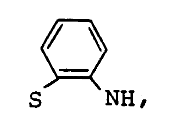

Y=CH₃, Z=COCH₃]:

Compound 27 (146 mg, yield 42%) was obtained as green powder from 299 mg of 1a-acetyl-7-demethoxy-6-demethyl-6,7-dihydro-7-ethylenedioxy-6-methylenemitomycin A, 90 mg of 2-aminothiophenol, 0.10 ml of triethylamine and 30 ml of dichloromethane in a similar manner as in Example 7.
TLC : R_{f}=0.59 (CHCl₃ :CH₃OH = 9 :1)
NMR (90MHz, CDCl₃) δ: major peaks, 8.94(bs, 1H), 7.6-6.9(m, 4H), 3.25(s, 3H), 2.13(s, 3H)

### Example 27

### Compound 28 [Compound (I): R¹=CH₂OCONH₂, R²=H, W-X=

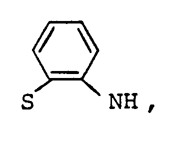

Y=CH₃, Z=H]:
Compound 27 (123 mg) obtained in Example 26 was dissolved in 50 ml of methanol, and 11 mg of potassium carbonate was added to the solution, followed by stirring at room temperature for 3 hours and 20 minutes. The reaction mixture was neutralized with phosphate buffer, pH 4, and extracted with chloroform. By post-treatment, purification and drying in a similar manner as in Example 22, 75.9 mg (yield 57%) of Compound 28 was obtained as green powder.
TLC : R_{f}=0.52 (CHCl₃ :CH₃OH = 9 :1)
MS (m/z): 441 (M⁺+1); C₂₁H₂₀N₄O₅S = 440
IR (cm⁻¹): 3450, 3350, 3300, 3200, 2920, 1710, 1640, 1570, 1520, 1480, 1330, 1240, 1150, 1070
NMR (270MHz, pyridine-d₅) δ: 9.35(s, 1H), 8.0-7.4(br, 2H), 7.48(dd, J=7.7, 1.5Hz, 1H), 7.36(dd, J=7.1, 1.1Hz, 1H), 7.26(dt, J=7.2, 1.4Hz, 1H), 7.03(dt, J=7.4, 1.4Hz, 1H), 5.37(dd, J=10.4, 4.2Hz, 1H), 5.10(t, J=10.8Hz, 1H), 4.45(d, J=12.8Hz, 1H), 4.19(d, J=15.6Hz, 1H), 4.03(dd, J=11.3, 4.3Hz, 1H), 3.84(d, J=15.4Hz, 1H), 3.59(d, J=12.8Hz, 1H), 3.23(s, 3H), 3.16(bs, 1H), 2.78(bs, 1H), 2.22(bt, 1H)

### Example 28

### 6-Demethyl-6-cyclopropylmethylthiomethylmitomycin C (Compound 29):

1a-Acetyl-7-demethoxy-6-demethyl-6,7-dihydro-7-ethylenedioxy-6-cyclopropylmethylthiomethylmitomycin A [Compound (III): R¹=CH₂OCONH₂, R²=H, 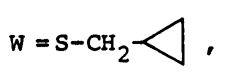
Y=CH₃, Z=COCH₂] (204 mg, yield 56%) was obtained from 300 mg of 1a-acetyl-7-demethoxy-6-demethyl-6,7-dihydro-7-ethylenedioxy-6-methylenemitomycin A, 100 µl of cyclopropylmethanethiol and 30 ml of dichloromethane in a similar manner as in Example 7.

Compound 29 (73.6 mg, yield 44%) was obtained as purple powder from 204 mg of the above compound, 30 ml of anhydrous tetrahydrofuran and ammonia gas in a similar manner as in Example 21.
TLC : R_{f}=0.34 (CHCl₃ :CH₃OH=9 :1)
MS (m/z): 421 (M⁺+1); C₁₉H₂₄N₄O₅S=420
IR (cm⁻¹): 3430, 3310, 3200, 2920, 1710, 1590, 1550, 1440, 1330, 1220, 1070
NMR (270MHz, pyridine-d₅) δ: 8.3-8.0(br, 2H), 7.9-7.4 (br, 2H), 5.42(dd, J=10.4, 4.2Hz, 1H), 5.10(bt, 1H), 4.57(d, J=12.8Hz, 1H), 4.04(d, J=12.8Hz, 1H), 4.03(dd, J=11.0, 4.9Hz, 1H), 3.86(d, J=13.0Hz, 1H), 3.61(d, J=11.9Hz, 1H), 3.19(s, 3H), 3.14(bs, 1H), 2.74(bs, 1H), 2.60(dd, J=13.0, 7.0Hz, 1H), 2.52(dd, J=12.9, 7.1Hz, 1H), 2.14(br, 1H), 1.10 (m, 1H), 0.43(m, 2H), 0.18(m, 2H)

### Reference Example 1

### 1a-Acetyl-7-demethoxy-6,7-dihydro-7-ethylenedioxymitomycin A [Compound (V): R¹=CH₂OCONH₂, R²=H, Y=CH₃, Z=COCH₃, n=2]:

Mitomycin A (1.0 g) was dissolved in 15 ml of tetrahydrofuran and 3 ml of ethylene glycol, and 0.5 ml of 1.6% (w/w) solution of potassium hydroxide in ethylene glycol was added to the solution. The mixture was stirred at 25°C for 5 hours, followed by addition of excess dry ice in small pieces. After stirring, the reaction mixture was diluted with chloroform. The organic layer was washed with saturated aqueous sodium chloride solution and dried over anhydrous sodium sulfate. The solvent was distilled off under reduced pressure and the residue was subjected to silica gel column chromatography. Elution was carried out with chloroform : methanol (97 : 3, v/v), and yellowish orange fractions were collected. After the solvent was distilled off under reduced pressure, the residue was dissolved in a small quantity of chloroform and n-hexane was added to the solution to give powder. Then, the solvent was distilled off under reduced pressure. The residue was thoroughly dried in vacuo at 25°C to give 880 mg (yield 81%) of 7-demethoxy-6,7-dihydro-7-ethylenedioxymitomycin A as yellowish orange powder.
TLC : R_{f}=0.27(CHCl₃ :CH₃OH = 9 :1)
MS (m/z): 380 (M⁺+1); C₁₇H₂₁N₃O₇ = 379
IR (cm⁻¹); 3446, 3296, 2902, 1727, 1702, 1642, 1575, 1447, 1336, 1186, 1068, 964, 855, 757, 705
NMR (400MHz, CDCl₃) δ: 0.90(bs, 1H), 1.18(d, J=6.6Hz, 3H), 2.80(bs, 1H), 2.91(d, J=4.2Hz, 1H), 3.21(s, 3H), 3.27(q, J=6.6Hz, 1H), 3.44(dd, J=12.3, 1.5 Hz, 1H), 3.62(dd, J=10.6, 4.4Hz, 1H), 3.83(d, J=12.8Hz, 1H), 3.98-4.13(m, 3H), 4.39(m, 1H), 4.58 (t, J=10.6Hz, 1H), 4.78(dd, J=10.6, 4.4Hz, 1H), 4.80(bs, 2H)

The above compound (52 mg) was dissolved in 1.0 ml of chloroform and 0.5 ml of pyridine, and 13 µl of acetic anhydride was added to the solution. After stirring at 25°C for one hour, 1.0 ml of methanol was added to the mixture, followed by stirring for 10 minutes. Then, 2 ml of toluene was added to the reaction mixture and the solvent was distilled off under reduced pressure. The residue was subjected to silica gel column chromatography. Elution was carried out with chloroform : methanol (96 : 4, v/v), and yellow fractions were collected. After the solvent was distilled off under reduced pressure, the residue was treated in a similar manner as above to give 53 mg (yield 95%) of the desired compound as yellow powder.

From the NMR spectrum, the obtained compound was found to be a diastereomeric mixture of about 2.5 : 1 having different stereochemistry at the C₆-position.
TLC : R_{f}=0.52 (CHCl₃ :CH₃OH=9 :1)
MS (m/z): 422 (M⁺+1); C₁₉H₂₃N₃O₈ = 421
IR (cm⁻¹): 3480, 3292, 2900, 1720, 1700, 1645, 1575, 1448, 1328, 1268, 1189, 1067, 1031, 949, 859, 749
NMR (400MHz, CDCl₃) δ:
Major: 1.20(d, J=6.6Hz, 3H), 2.11(s, 3H), 3.21(s, 3H), 3.22(q, J=6.6Hz, 1H), 3.23(dd, J=4.4, 2.0Hz, 1H), 3.47(dd, J=13.1, 2.0Hz, 1H), 3.50(d, J=4.4 Hz, 1H), 3.73(dd, J=10.8, 4.9Hz, 1H), 4.04(d, J=13.1Hz, 1H), 3.98-4.41(M, 4H), 4.17(t, J=11.1Hz, 1H), 4.82(bs, 2H), 4.98(dd, J=11.1, 4.9Hz, 1H)
Minor: major peaks
1.24(d, J=6.9Hz, 3H), 2.11(s, 3H), 3.04(q, J=6.9Hz, 1H), 3.22(s, 3H), 3.42(dd, J=13.0, 1.7Hz, 1H), 4.34(d, J=13.0Hz, 1H), 4.89(dd, J=10.8, 4.9 Hz, 1H)

### Reference Example 2

### 1a-Acetyl-7-demethoxy-6,7-dihydro-7-ethylenedioxy-6-phenylselenomitomycin A [Compound (VI): R¹=CH₂OCONH₂, R²=H, Y=CH₃, Z=COCH₃, n=2]:

1a-Acetyl-7-demethoxy-6,7-dihydro-7-ethylenedioxymitomycin A (120 mg) was dissolved in 4.0 ml of tetrahydrofuran, and 0.3 ml of triethylamine was added to the solution. After stirring at 25°C for 30 minutes, 67 mg of phenylselenenyl bromide was added to the mixture. The mixture was stirred at 25°C for 30 minutes and 60 mg of phenylselenenyl bromide was further added. After stirring for 20 minutes, the solvent was distilled off under reduced pressure and the residue was subjected to silica gel column chromatography. Elution was carried out with chloroform : methanol (97 : 3, v/v), and yellow fractions were collected. After the solvent was distilled off under reduced pressure, the residue was treated in a similar manner as in Reference Example 1 to give 149 mg (yield 91%) of the desired compound as yellow powder.

From the NMR spectrum, the obtained compound was found to be a diastereomeric mixture of about 2 : 1 having different stereochemistry at the C₆-position.
TLC : R_{f}=0.49 (CHCl₃ :CH₃OH=9 :1)
MS (m/z): 578 (M⁺+2); C₂₅H₂₇N₃O₈Se=576
IR (cm⁻¹): 3460, 2970, 1725, 1688, 1656, 1571, 1435, 1377, 1334, 1247, 1205, 1066, 1031, 987, 857, 742
NMR (400MHz, CDCl₃) δ:
Major: 1.45(s, 3H), 2.18(s, 3H), 3.16(s, 3H), 3.27(dd, J=4.7, 2.0Hz, 1H), 3.39(dd, J=13.0, 2.0Hz, 1H), 3.51(d, J=4.4Hz, 1H), 3.80(dd, J=11.3, 4.9Hz, 1H), 3.84(d, J=13.0Hz, 1H), 3.95-4.50(m, 4H), 4.28(t, J=11.1Hz, 1H), 5.15(dd, J=11.0, 4.7Hz, 1H), 5.80(bs, 2H), 7.24-7.63(m, 5H)
Minor: major peaks
1.42(s, 3H), 2.18(s, 3H), 3.22(dd, J=4.4, 2.0Hz, 1H), 3.26(s, 3H), 3.38(dd, J=13.0, 2.2Hz, 1H), 3.47(d, J=4.4Hz, 1H), 3.75(dd, J=11.0, 4.7Hz, 1H), 4.14(t, J=10.8Hz, 1H), 4.54(d, J=12.9Hz, 1H), 4.85(dd, J=11.0, 4.7Hz, 1H)

### Reference Example 3

### 1a-Acetyl-7-demethoxy-6-demethyl-6,7-dihydro-7-ethylenedioxy-6-methylenemitomycin A [Compound (II): R¹=CH₂OCONH₂, R²=H, Y=CH₃, Z=COCH₃, n=2]:

1a-Acetyl-7-demethoxy-6,7-dihydro-7-ethylenedioxy-6-phenylselenomitomycin A (833 mg) was dissolved in 30 ml of chloroform, and 380 mg of potassium carbonate was added to the solution. The mixture was cooled to 0°C and 17 ml of a solution of 425 mg of MCPBA in chloroform was added dropwise thereto over 40 minutes. After stirring at room temperature for 50 minutes, the reaction mixture was poured into an aqueous solution mixture of sodium thiosulfate and sodium bicarbonate. The resulting mixture was extracted with chloroform. The chloroform layer was washed with saturated aqueous sodium chloride solution and then dried over anhydrous sodium sulfate, followed by addition of n-hexane to give powder. The solvent was distilled off under reduced pressure and the residue was thoroughly dried in vacuo to give 590 mg (yield 97%) of the desired compound as yellow powder.
TLC: R_{f}=0.52 (CHCl₃:CH₃OH=9:1)
MS (m/z): 420 (M⁺ +1); C₁₉H₂₁N₃O₈=419
IR (cm⁻¹): 3470, 3370, 2950, 1702, 1658, 1571, 1458, 1383, 1333, 1269, 1110, 1057, 1025, 984, 948, 861, 801, 709
NMR (400MHz, CDCl₃) δ: 6.39(d, J=1.2Hz, 1H), 6.11(d,J=1.2Hz, 1H), 4.92(dd, J=10.8, 4. 7Hz, 1H), 4.77 (b, 2H), 4.39(4, J=13.0Hz, 1H), 4.32-4.08(m, 4H), 4.15(t, J=10.8Hz, 1H), 3.75(dd, J=10.8, 4.9Hz, 1H), 3.52(dd, J=13.3, 2.0Hz, 1H), 3.51(d, J=4.4 Hz, 1H), 3.24(dd, J=4.4, 2.0Hz, 1H), 3.22(s, 3H), 2.12(s, 3H)
Without prejudice to any Registered Trade Marks used herein, but not specifically acknowledged as such, the following are acknowledged as being Registered Trade Marks in one or more of the designated states:
TRITON
TWEEN

## Claims

1. Mitomycin derivatives of the formula 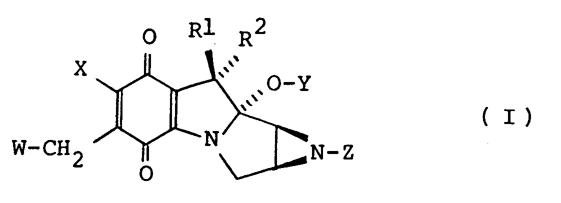 wherein:
W is an RO or RS group, where R is H; straight or branched chain C₁-C₁₂ alkyl, optionally containing one or two substituents selected from: OH, C₁-C₄ alkoxy, C₂-C₅ alkoxycarbonyl, C₁-C₄ alkanoylamino, C₁-C₄ alkanoyloxy, aroylamino or aroyloxy, wherein the aroyl group is benzoyl, toluoyl, p-nitrobenzoyl or naphthoyl, halogen and C₃-C₆ cycloalkyl; C₂-C₁₂ alkenyl, optionally containing one or two substituents selected from: OH, C₁-C₄ alkoxy, C₂-C₅ alkoxycarbonyl, and halogen; phenyl, naphthyl, benzyl, phenethyl or benzhydryl, optionally containing from 1 - 3 substituents in the aryl moiety and selected from: OH, C₁-C₄ alkoxy, C₂-C₅ alkoxycarbonyl, C₁-C₄ alkanoylamino, C₁-C₄ alkanoyloxy, aroylamino or aroyloxy, wherein the aroyl group is benzoyl, toluoyl, p-nitrobenzoyl or naphthoyl, halogen, nitro and C₁-C₄ alkyl;
X is methoxy or amino;
Y is H or methyl;
Z is H, methyl, C₁-C₄ alkanoyl, benzoyl, toluyl, p-nitrobenzoyl, or naphthoyl; and
one of R¹ and R² represents carbamoyloxymethyl and the other represents hydrogen, or together they jointly represent a methylene (=CH₂) group;
or wherein:
W and X together form either an -S(CH₂)₂NH- group or a group of the formula:

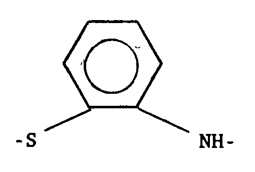

2. Mitomycin derivatives according to claim 1, wherein W is an RO or RS group in which R is methyl, ethyl, n-propyl, isopropyl, n-butyl, isobutyl, tert-butyl, pentyl, hexyl, heptyl, octyl, decyl or dodecyl.

3. Mitomycin derivatives according to claim 1, wherein W is an RO or RS group in which R is vinyl, allyl, homoallyl, crotyl or cis-7-dodecenyl.

4. Mitomycin derivatives of the formula defined in claim 1, being those specific compounds where W, X, Y, Z, R¹ and R² have the values tabulated below and herein identified as Compounds Nos. 1 - 29:

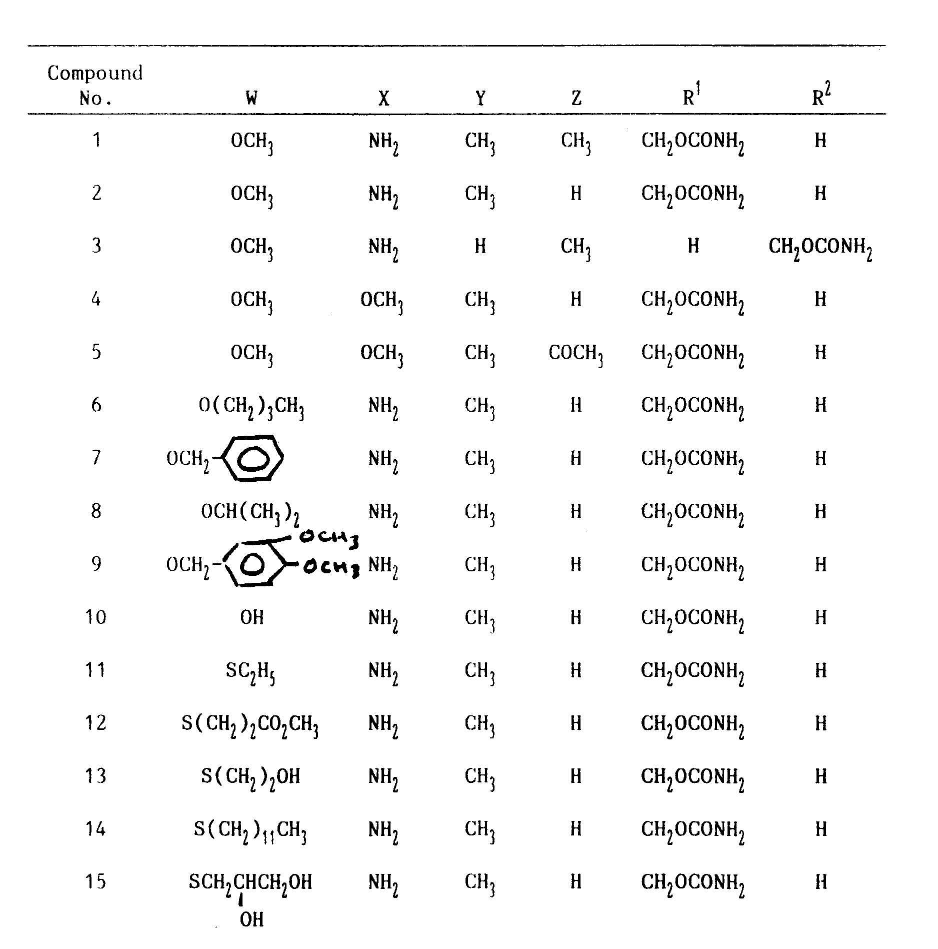

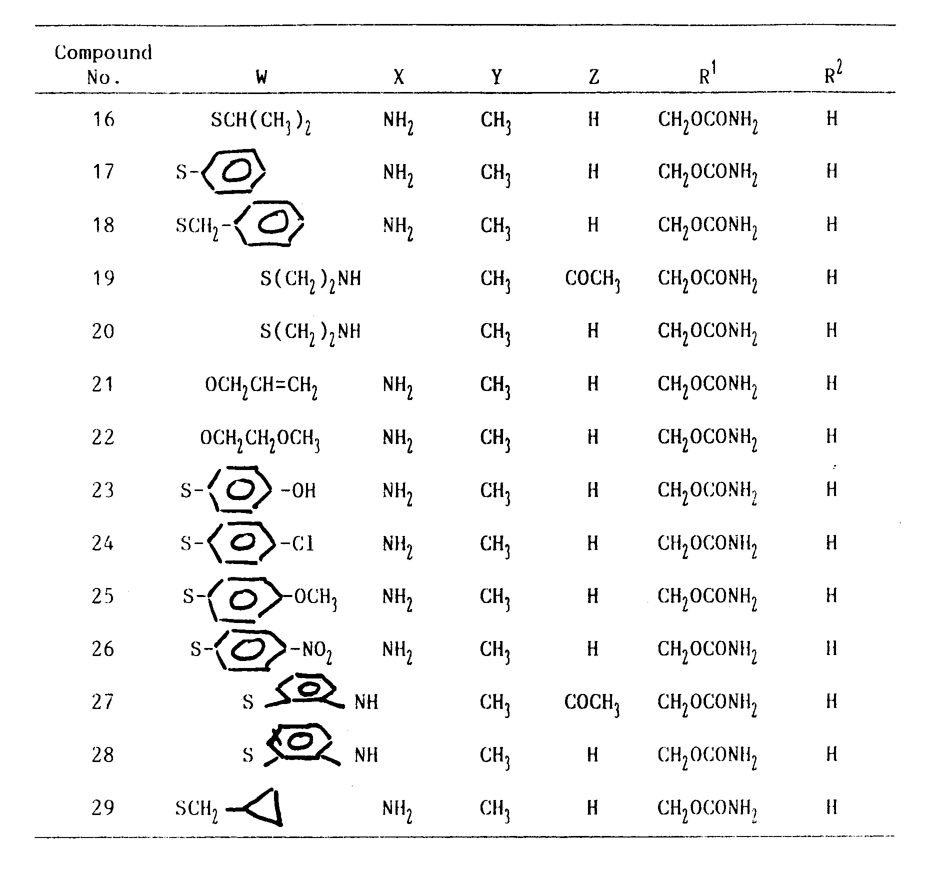

5. A pharmaceutical composition comprising a mitomycin derivative of the formula defined in claim 1, in admixture with a pharmaceutically acceptable carrier or diluent.

6. A composition according to claim 5, wherein the mitomycin derivative is a compound as claimed in any one of claims 2 to 4.

## Patentansprüche

1. Mitomycin-Derivate der Formel 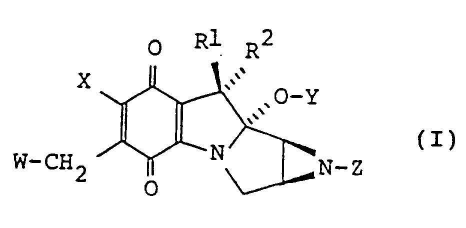 in der
W ist: ein RO- oder RS-Rest, wobei R ein H-Atom ist; eine geradkettige oder verzweigte C₁-C₁₂-Alkylgruppe, die gegebenenfalls einen oder zwei Substituenten enthält, die ausgewählt sind aus einer OH-Gruppe, C₁-C₄-Alkoxy-, C₂-C₅-Alkoxy-carbonyl-, C₁-C₄-Alkanoylamino-, C₁-C₄-Alkanoyloxygruppe, einem Aroylamino- oder Aroyloxyrest, wobei der Aroylrest eine Benzoyl-, Toluoyl-, p-Nitrobenzoyl- oder Naphthoylgruppe, ein Halogenatom und eine C₃-C₆-Cycloalkylgruppe ist; eine C₂-C₁₂-Alkenylgruppe, die gegebenenfalls einen oder zwei Substituenten enthält, die ausgewählt sind aus: einer OH-Gruppe, C₁-C₄-Alkoxy-, C₂-C₅-Alkoxycarbonylgruppe und einem Halogenatom; eine Phenyl-, Naphthyl-, Benzyl-, Phenethyl- oder Benzhydrylgruppe, die gegebenenfalls 1 bis 3 Substituenten in der Aryleinheit enthält, die ausgewählt sind aus: einer OH-Gruppe, C₁-C₄-Alkoxy-, C₂-C₅-Alkoxycarbonyl-, C₁-C₄-Alkanoylamino-, C₁-C₄-Alkanoyloxygruppe, einem Aroylamino- oder Aroyloxyrest, wobei der Aroylrest eine Benzoyl-, Toluoyl-, p-Nitrobenzoyl oder Naphthoylgruppe, ein Halogenatom, eine Nitro- und eine C₁-C₄-Alkylgruppe ist;
X eine Methoxy- oder Aminogruppe ist;
Y ein H-Atom oder eine Methylgruppe ist;
Z ein H-Atom, eine Methyl-, C₁-C₄-Alkanoyl-,
Benzoyl-, Toluyl-, p-Nitrobenzoyl- oder Naphthoylgruppe ist; und
einer der Reste R¹ und R² eine Carbamoyloxymethylgruppe und der andere ein Wasserstoffatom ist, oder diese miteinander Verbunden eine Methylengruppe (=CH₂) sind;
oder in der:
W und X miteinander entweder eine -S(CH₂)₂NH-Gruppe oder eine Gruppe der Formel bilden:

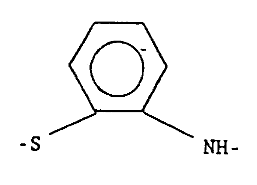

2. Mitomycin-Derivate nach Anspruch 1, wobei W ein RO- oder RS-Rest ist, indem R eine Methyl-, Ethyl-, n-Propyl-, Isopropyl-, n-Butyl-, Isobutyl-, tert.Butyl-, Pentyl-, Hexyl-, Heptyl-, Octyl-, Decyl- oder Dodecylgruppe ist.

3. Mitomycin-Derivate nach Anspruch 1, wobei W ein RO- oder RS-Rest ist, in dem R eine Vinyl-, Allyl-, Homoallyl-, Crotyl- oder cis-7-Dodecenylgruppe ist.

4. Mitomycin-Derivate der in Anspruch 1 definierten Formel, die bestimmte Verbindungen sind, in denen W, X, Y, Z, R¹ und R² die nachstehend aufgelisteten Bedeutungen haben und hier als Verbindungen Nr. 1 - 29 bezeichnet werden:

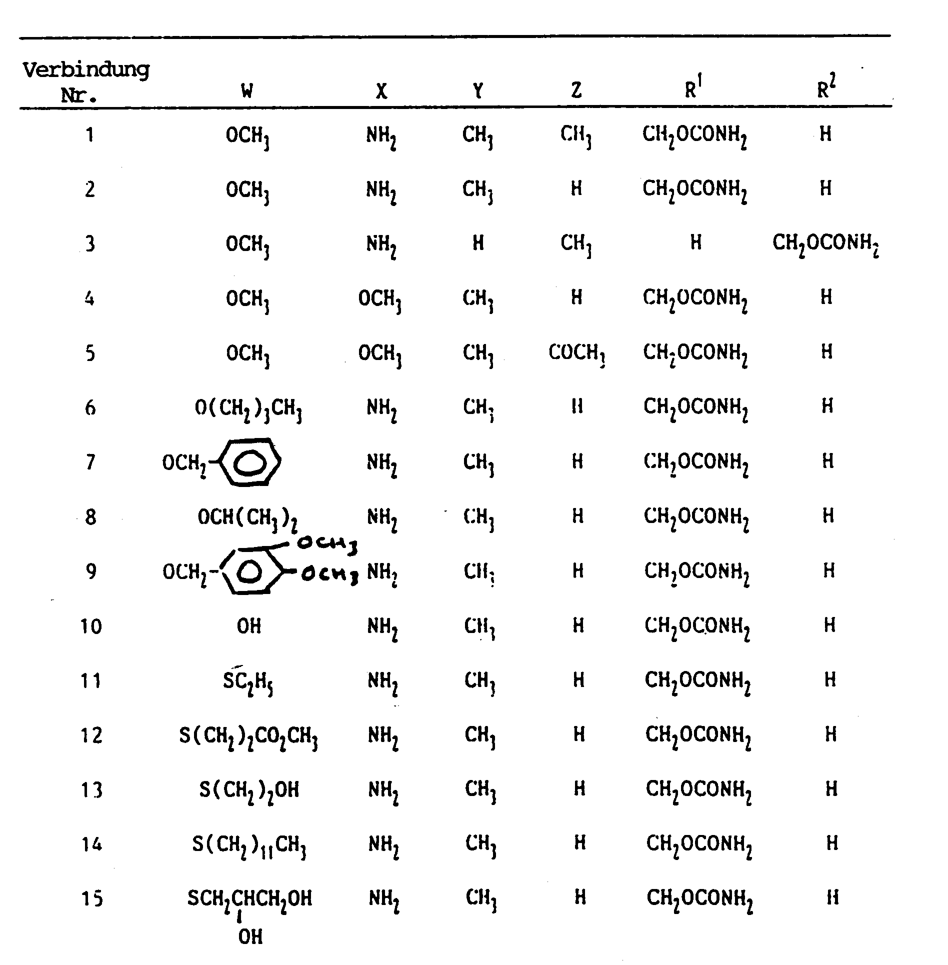

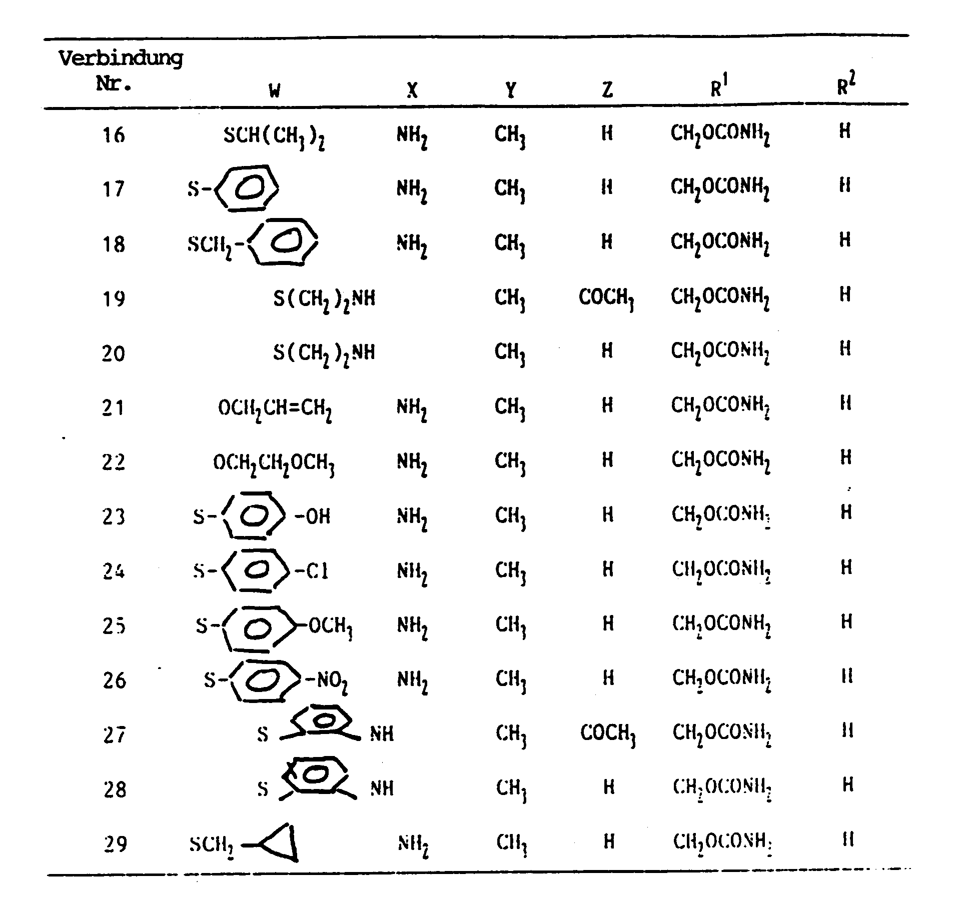

5. Arzneimittel, umfassend ein Mitomycin-Derivat der in Anspruch 1 definierten Formel in Vermischung mit einem pharmazeutisch verträglichen Träger oder Verdünnungsmittel.

6. Mittel nach Anspruch 5, wobei das Mitomycin-Derivat eine Verbindung nach einem der Ansprüche 2 bis 4 ist.

## Revendications

1. Dérivés de mitomycine de formule : 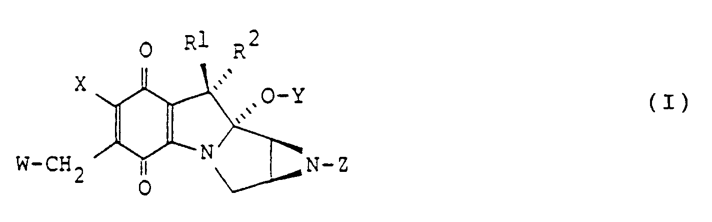 dans laquelle
W représente un groupe RO ou RS, R représentant un atome d'hydrogène ; un groupe alkyle en C₁-C₁₂ à chaîne droite ou ramifiée, portant éventuellement un ou deux substituants choisis parmi hydroxy, alcoxy en C₁-C₄, alcoxycarbonyle en C₂-C₅, alcanoylamino en C₁-C₄, alcanoyloxy en C₁-C₄, aroylamino ou aroyloxy, le groupe aroyle représentant un groupe benzoyle, toluyle, p-nitrobenzoyle ou naphtoyle, halogéno et cycloalkyle en C₃-C₆ ; un groupe alcényle en C₂-C₁₂, portant éventuellement un ou deux substituants choisis parmi hydroxy, alcoxy en C₁-C₄, alcoxycarbonyle en C₂-C₅ et halogéno ; ou un groupe phényle, naphtyle, benzyle, phénéthyle ou benzhydryle, dont le fragment aryle porte éventuellement de 1 à 3 substituants choisis parmi hydroxy, alcoxy en C₁-C₄, alcoxycarbonyle en C₂-C₅, alcanoylamino en C₁-C₄, alcanoyloxy en C₁-C₄, aroylamino ou aroyloxy, le groupe aroyle étant un groupe benzoyle, toluoyle, p-nitrobenzoyle ou naphtoyle, halogéno, nitro et alkyle en C₁-C₄ ;
X représente un groupe méthoxy ou amino ;
Y représente un atome d'hydrogène ou un groupe méthyle ;
Z représente un atome d'hydrogène ou un groupe méthyle, alcanoyle en C₁-C₄, benzoyle, toluyle, p-nitrobenzoyle ou naphtoyle ; et
l'un des R¹ et R² représente un groupe carbamoyloxyméthyle et l'autre représente un atome d'hydrogène, ou bien tous deux représentent conjointement un groupe méthylène (=CH₂) ;
ou dans laquelle
W et X forment ensemble soit un groupe -S(CH₂)₂NH-, soit un groupe de formule :

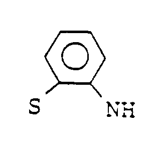

2. Dérivés de mitomycine conformes à la revendication 1, dans lesquels W représente un groupe RO ou RS dans lequel R représente un groupe méthyle, éthyle, n-propyle, isopropyle, n-butyle, isobutyle, tertiobutyle, pentyle, hexyle, heptyle, octyle, décyle ou dodécyle.

3. Dérivés de mitomycine conformes à la revendication 1, dans lesquels W représente un groupe RO ou RS dans lequel R représente un groupe vinyle, allyle, homoallyle, crotyle ou cis-7-dodécényle.

4. Dérivés de mitomycine de formule définie dans la revendication 1, qui sont les composés particuliers dans lesquels W, X, Y, Z, R¹ et R² ont les significations indiquées dans le tableau ci-dessous, dans lequel ils sont identifiés comme étant les Composés N° 1 à 29 :

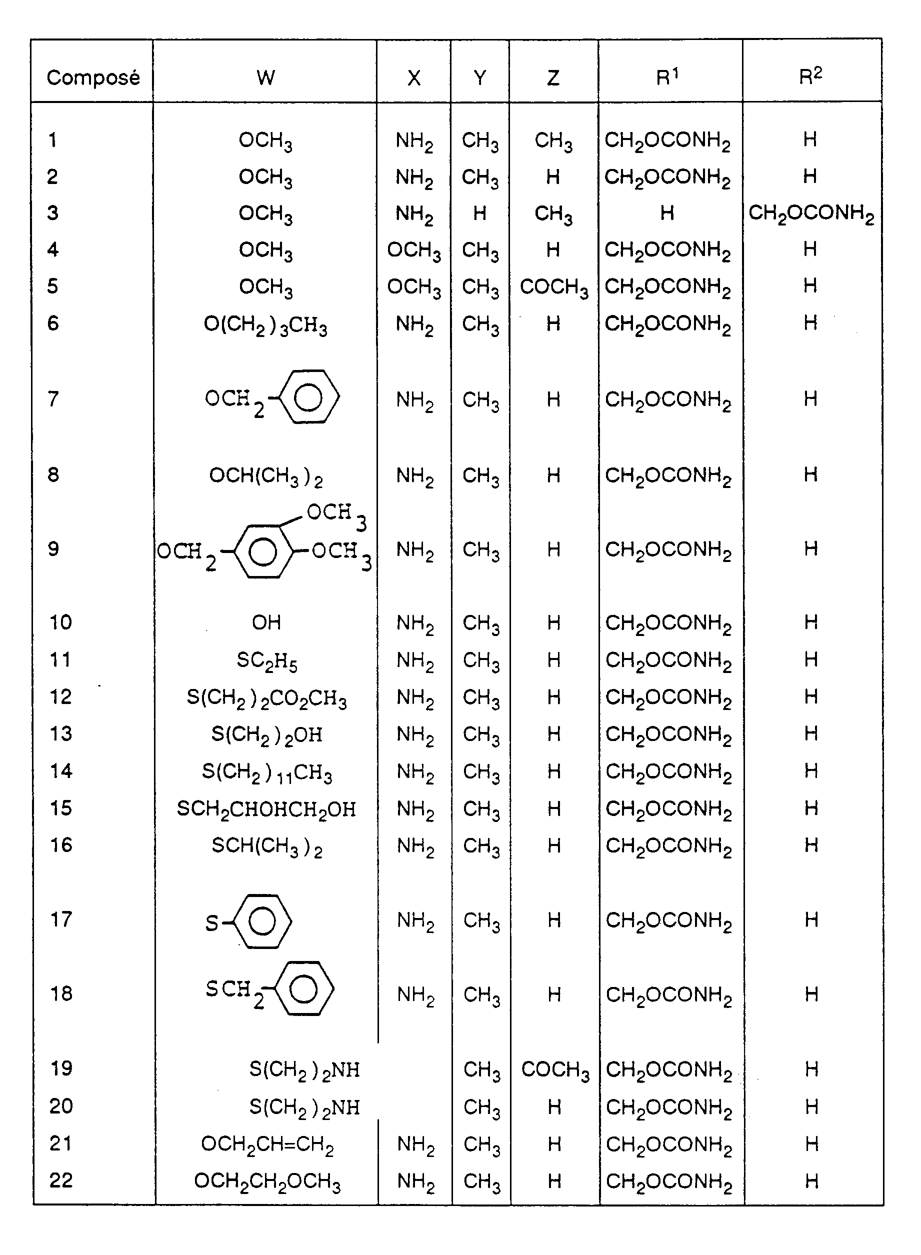

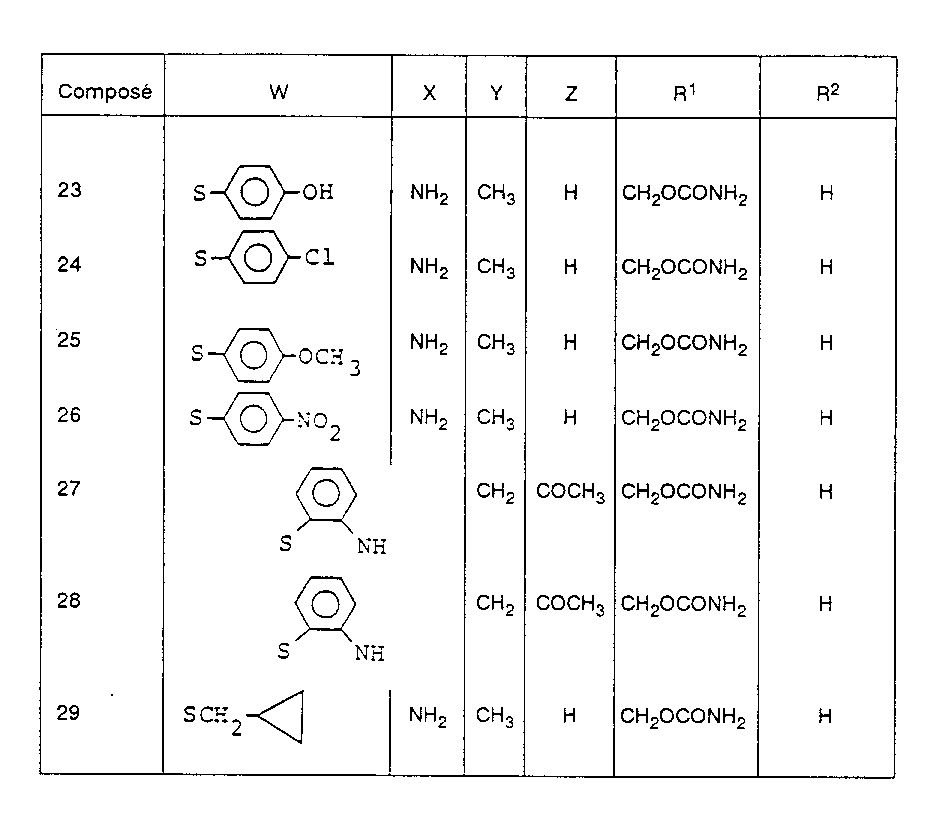

5. Composition pharmaceutique comprenant un dérivé de mitomycine de formule définie dans la revendication 1, mélangé avec un véhicule ou diluant pharmaceutiquement acceptable.

6. Composition conforme à la revendication 5, dans laquelle le dérivé de mitomycine est un composé revendiqué dans l'une quelconque des revendications 2 à 4.
